# FASCICULE DE BREVET EUROPEEN

(11) **EP 0 738 263 B1**
(45) Date de publication et mention de la délivrance du brevet: **04.04.2001**
(21) Numéro de dépôt: 95906361.1
(22) Date de dépôt: 04.01.1995
(51) Int. Cl.: C07D 233/78, C07D 235/02, A61K 31/415

(54) **PHENYLIMIDAZOLIDINES SUBSTITUEES A ACTIVITE ANTIANDROGENE**
SUBSTITUERTE PHENYLIMIDAZOLIDINE MIT ANTIANDROGENER AKTIVITÄT
SUBSTITUTED PHENYLIMIDAZOLIDINES HAVING ANTIANDROGEN ACTIVITY

(30) Priorité: 05.01.1994 FR 9400042; 06.09.1994 FR 9410660
(43) Date de publication de la demande: 23.10.1996
(73) Titulaire: Aventis Pharma S.A., 92160 Antony (FR)
(72) Inventeur: CLAUSSNER, André, F-93250 Villemomble (FR); GOUBET, François, F-75015 Paris (FR); TEUTSCH, Jean-Georges, F-93500 Pantin (FR)
(86) Numéro de dépôt international: FR9500004
(87) Numéro de publication internationale: WO9518794

(56) Documents cités:
- EP-A- 0 001 813
- EP-A- 0 017 976
- EP-A- 0 091 596
- EP-A- 0 305 270
- EP-A- 0 494 819
- EP-A- 0 578 516
- EP-A- 0 580 459
- BE-A- 884 897
- DE-A- 2 540 872
- DE-B- 1 032 258
- FR-A- 2 024 141
- FR-A- 2 117 527
- FR-A- 2 329 276
- GB-A- 997 037
- US-A- 4 093 444
- US-A- 4 753 957
- PATENT ABSTRACTS OF JAPAN vol. 2 no. 15 (C-77) ,31 Janvier 1978 & JP,A,52 113965 (NIPPON SODA K.K.) 24 Septembre 1977,
- PATENT ABSTRACTS OF JAPAN vol. 2 no. 148 (C-78) ,9 Décembre 1978 & JP,A,53 112875 (SUMITOMO KAGAKU KOGYO K.K.) 2 Octobre 1978,
- PATENT ABSTRACTS OF JAPAN vol. 3 no. 3 (C-33) ,16 Janvier 1979 & JP,A,53 124267 (SUMITOMO KAGAKU KOGYO K.K.) 30 Octobre 1978,
- PATENT ABSTRACTS OF JAPAN vol. 3 no. 50 (C-44) ,27 Avril 1979 & JP,A,54 027564 (SUMITOMO KAGAKU KOGYO K.K.) 1 Mars 1979,

## Description

La présente invention concerne de nouvelles phénylimidazolidines éventuellement substituées, leur procédé et des intermédiaires de préparation, leur application comme médicaments et les compositions pharmaceutiques les renfermant.

Dans la demande japonaise J 48087030 sont décrites des 3-phényl 2-thiohydantoïnes qui sont présentées comme inhibant la germination de certaines plantes.

Dans les demandes de brevets européens 0 494 819 et 0 578 516 sont décrites des imidazolidines qui sont présentées comme possédant une activité antiandrogène. Les produits de ce brevet sont cependant différents des produits de la présente demande de brevet.

Le brevet FR 2329276 décrit des imidazolidines possédant une activité anti-androgène.

Le brevet EP 0305270 décrit des imidazolidines substituées par un radical hydroxyméthyle possédant une activité anti-androgène.

Le brevet EP 0580 459 décrit des phénylimidazolidines substituées possédant une activité anti-androgène.

La présente invention a donc pour objet les produits de formule générale (I) : 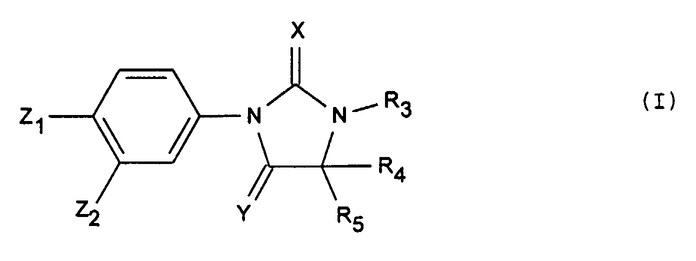 dans laquelle :
Y représente un atome d'oxygène ou un radical NH, Z₂ représente un radical trifluorométhyle et Z₁ représente un radical cyano ou nitro,
X représente un atome d'oxygène ou de soufre,
R₃ représente un atome d'hydrogène ou un radical alkyle ayant au plus 4 atomes de carbone, éventuellement substitué par un ou plusieurs radicaux choisis parmi les atomes d'halogène ou le radical cyano,
R₄ et R₅ sont tels que :
soit l'un représente méthyle et l'autre est choisi parmi les valeurs de R₄ et R₅ ci-après,
soit R₄ et R₅ identiques ou différents représentent un radical alkyle linéaire ou ramifié renfermant au plus 4 atomes de carbone substitué par un radical hydroxyle éventuellement substitué par un radical phényle, alkyle renfermant au plus 4 atomes de carbone ou acyle renfermant au plus 7 atomes de carbone ; un atome d'halogène ; ou un radical phénylthio éventuellement substitué par un atome d'halogène ou un radical hydroxyle,
étant entendu que R4 et R5 ne représentent pas l'un méthyle et l'autre hydroxyméthyle ou tertiobutyloxymethyle lorsque Z1 représente nitro, Z2 représente CF3, R3 représente hydrogène, X représente oxygène et Y représente oxygène ou NH,
lesdits produits de formule (I) étant sous toutes les formes isomères racémiques, énantiomères et diastéréoisomères possibles, ainsi que les sels d'addition avec les acides minéraux et organiques ou avec les bases minérales et organiques desdits produits de formule (I).

Pour la définition des substituants indiqués ci-dessus et dans ce qui suit, les définitions utilisées peuvent avoir les valeurs suivantes :

Le terme alkyle ayant au plus 4 atomes de carbone désigne un radical linéaire ou ramifié méthyle, éthyle, propyle, isopropyle, butyle, isobutyle, secbutyle, ou tert-butyle.

On préfère les radicaux méthyle, éthyle, propyle, isopropyle et n-butyle.

Par halogène, on entend bien entendu, les atomes de fluor, de chlore, de brome ou d'iode.

On préfère les atomes de fluor, de chlore ou de brome.

Comme exemples particuliers de radicaux alkyle substitués par un ou plusieurs halogènes, on peut citer les radicaux monofluoro, chloro, bromo ou iodométhyle, difluoro, dichloro ou dibromométhyle et trifluorométhyle.

Par radical acyle ayant au plus 7 atomes de carbone, on entend de préférence un radical tel que le radical acétyle, propionyle, butyryle ou benzoyle, mais peut également représenter un radical valéryle, hexanoyle, acryloyle, crotonoyle ou carbamoyle : on peut également citer le radical formyle.

Des exemples de groupement protecteur de OH P, ainsi que la formation du radical hydroxyle protégé, sont donnés notamment dans le livre usuel de l'homme du métier : Protective Groups in Organic Synthesis, Theodora W. Greene, Harvard University, imprimé en 1981 par Wiley-Interscience Publishers, John Wiley & Sons.

Le groupement de protection du radical hydroxyle que peut représenter P, peut être choisi dans la liste ci-dessous :
par exemple formyle, acétyle, chloroacétyle, bromoacétyle, dichloroacétyle, trichloroacétyle, trifluoroacétyle, méthoxyacétyle, phénoxyacétyle, benzoyle, benzoylformyle, p-nitro-benzoyle. On peut citer également les groupements éthoxycarbonyle, méthoxycarbonyle, propoxycarbonyle, βββ-trichloro-éthoxycarbonyle, benzyloxycarbonyle, tert-butoxycarbonyle, 1-cyclo propyléthoxycarbonyle, tétrahydropyrannyle, tétrahydrothiopyrannyle, méthoxytétrahydropyrannyle, trityle, benzyle, 4-méthoxybenzyle, benzhydryle, trichloroéthyle, 1-méthyl 1-méthoxyéthyle, phtaloyle, propionyle, butyryle, isobutyryle, valéryle, isovaléryle, oxalyle, succinyle et pivaloyle, phénylacétyle, phénylpropionyle, mésyle, chlorobenzoyle, para-nitrobenzoyle, para-tert-butylbenzoyle, caprylyle, acryloyle, méthylcarbamoyle, phénylcarbamoyle, naphtylcarbamoyle.

P peut notamment représenter le radical 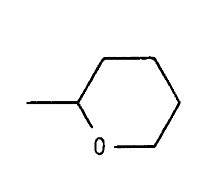 ou encore un dérivé du silicium tel que triméthylsilyle.

Lorsque les produits de formules (I) telle que définie ci-dessus comportent un radical amino salifiable par un acide il est bien entendu que ces sels d'acides font également partie de l'invention. On peut citer les sels formés avec les acides chlorhydrique ou méthanesulfonique par exemple.

Parmi les produits préférés de l'invention, on peut citer plus précisément les produits de formule (I) telle que définie ci-dessus dont les noms suivent :
- 2-(trifluorométhyl) 4-(4-(hydroxyméthyl) 4-méthyl 2,5-dioxo 1-imidazolidinyl) benzonitrile,
- 4-(3,4-diméthyl) 4-(hydroxyméthyl) 5-oxo 2-thioxo 1-imidazolidinyl) 2-(trifluorométhyl) benzonitrile,
- 2-(trifluorométhyl) 4-(4-(hydroxyméthyl) 3,4-diméthyl 2,5-dioxo 1-imidazolidinyl) benzonitrile,
- 4-(2,5-dioxo 3-(2-fluoroéthyl) 4-(hydroxyméthyl) 4-méthyl 1-imidazolidinyl) 2-(trifluorométhyl) benzonitrile,
- 4- (4,4-bis (hydroxyméthyl)-2,5-dioxo-1-imidazolidinyl) -2-(trifluorométhyl)-benzonitrile,
- 4- (4- (fluorométhyl) 3,4-diméthyl) 2,5-dioxo 1-imidazolidinyl) 2-(trifluorométhyl) benzonitrile,
- 4-(3,4-diméthyl) 4-(fluorométhyl) 5-oxo 2-thioxo 1-imidazolidinyl) 2-(trifluorométhyl) benzonitrile,
- 4-(2,5-dioxo 3-(2-fluoroéthyl) 4-(fluorométhyl) 4-méthyl 1-imidazolidinyl) 2-(trifluorométhyl) benzonitrile,
- 1,5-diméthyl 5-(fluorométhyl) 3-(4-nitro 3-(trifluorométhyl) phényl) 2,4-imidazolidinedione,
- 3-(4-cyano 3-(trifluorométhyl) phényl) 2,4-dioxo 5-(fluorométhyl) 5-méthyl 1-imidazolidinacétonitrile,
- 4-(4,4-bis-(fluorométhyl) 3-méthyl 5-oxo 2-thioxo 1-imidazolidinyl 2-(trifluorométhyl) benzonitrile.

L'invention a aussi pour objet un procédé de préparation des produits de formule (I), caractérisé en ce que :
soit l'on fait agir en présence d'une base tertiaire un produit de formule (II) : 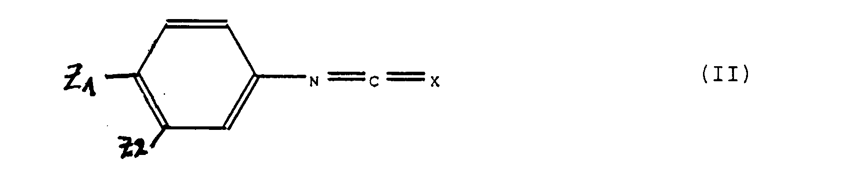 dans laquelle Z₁, Z₂ et X ont la signification indiquée ci-dessus, avec un produit de formule (III) : 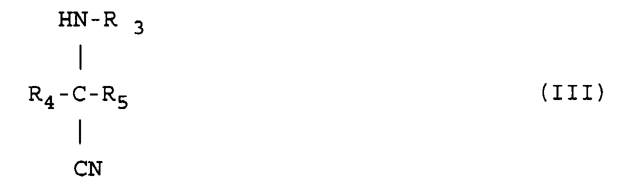 dans laquelle R₃, R₄ et R₅ ont la signification indiquée ci-dessus pour obtenir un produit de formule (IV) : 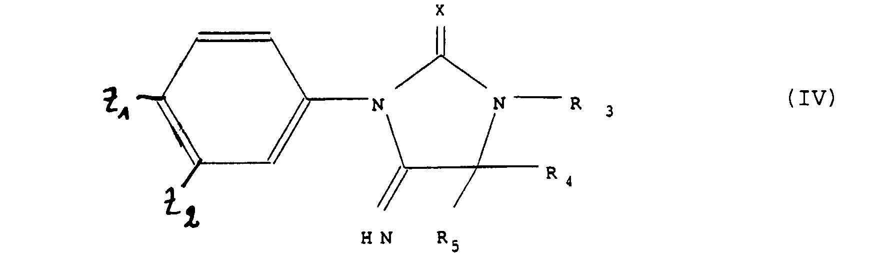 dans laquelle Z₁, Z₂, X, R ₃, R₄ et R₅ ont la signification précédente,
soit l'on fait agir en présence d'une base tertiaire le produit de formule (II), telle que définie ci-dessus, avec un produit de formule (VII) : 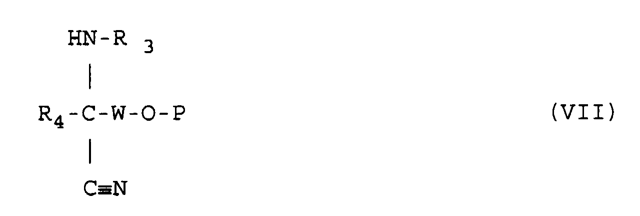 dans laquelle W a la signification indiquée ci-dessus pour R₅ à l'exception du radical alkyle substitué par un radical hydroxyle libre, ou substitué par un radical phényle, alkyle renfermant au plus 4 atomes de carbone ou acyle renfermant au plus 7 atomes de carbone et P représente un groupement protecteur de OH et R ₃ et R₄ ont la signification indiquée ci-dessus, pour obtenir un produit de formule (VIII) : 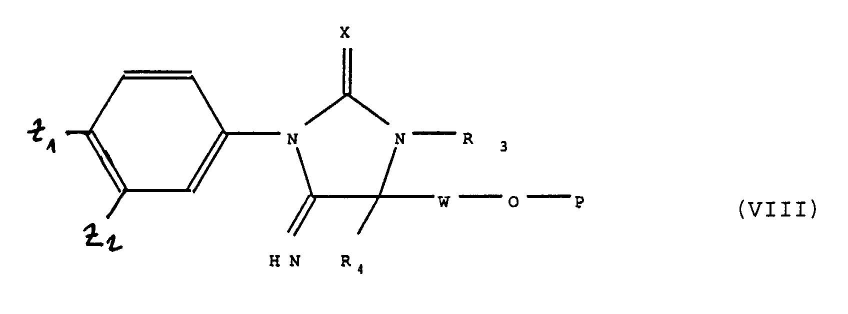 dans laquelle X, Z₁, Z₂, R₃, R₄, W et P ont la signification indiquée ci-dessus,
   produit de formule (VIII) dont si nécessaire et si désiré, l'on peut libérer de OP le radical OH que l'on peut alors si nécessaire et si désiré, estérifier ou transformer en radical halogène, produits de formules (IV) et (VIII) que, si nécessaire ou si désiré l'on soumet à l'une quelconque ou plusieurs des réactions suivantes, dans un ordre quelconque :
   b) réaction d'hydrolyse du groupement >C=NH en fonction carbonyle et le cas échéant transformation du groupement >C=S en groupement >C=O ;
   c) réaction de transformation du groupement >C=O en groupement >C=S ;
   d) action sur les produits de formule (IV) ou (VIII) dans laquelle R ₃ représente un atome d'hydrogène, et après hydrolyse du groupement >C=NH en fonction carbonyle d'un réactif de formule Hal-R"₃ dans laquelle R"₃ a les valeurs de R ₃ à l'exception de la valeur hydrogène et Hal représente un atome d'halogène pour obtenir des produits de formule (I) tels que définis ci-dessus,
soit l'on fait agir en présence d'une base tertiaire un produit de formule (II) tel que défini ci-dessus, avec un produit de formule (III') : 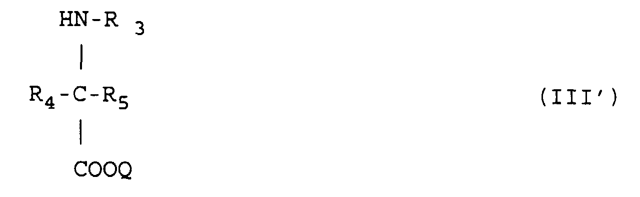 dans laquelle R ₃, R₄ et R₅ ont la signification indiquée ci-dessus et Q représente soit un atome de métal alcalin ou un radical alkyle renfermant de 1 à 6 atomes de carbone, pour obtenir un produit de formule (IVa) : 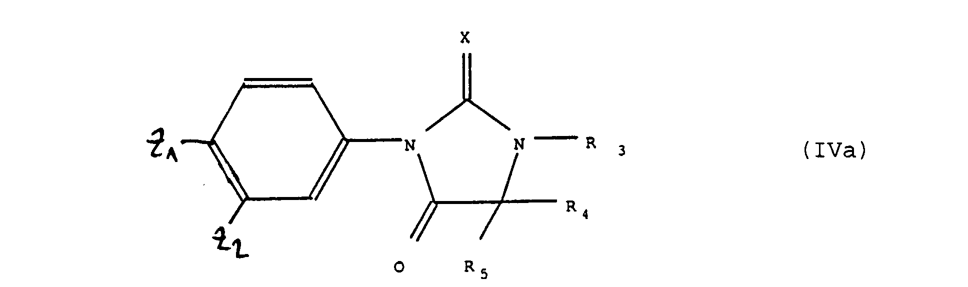 dans laquelle X, Z₁, Z₂, R ₃, R₄ et R₅ ont la signification indiquée ci-dessus, que si désiré l'on soumet à l'une quelconque ou plusieurs des réactions suivantes, dans un ordre quelconque :
   b) réaction de transformation du groupement >C=O en groupement >C=S ou le cas échéant du groupement >C=S en groupement >C=O ;
   c) action sur les produits de formule (IVa) dans laquelle R ₃ représente un atome d'hydrogène, d'un réactif de formule Hal-R"₃ dans laquelle R"₃ a les valeurs de R ₃ à l'exception de la valeur hydrogène et Hal représente un atome d'halogène pour obtenir des produits de formule (I) tels que définis ci-dessus,
soit l'on fait agir un réactif de formule Hal-R"₃ dans laquelle Hal et R"₃ ont les valeurs indiquées précédemment sur un produit de formule (IV') : 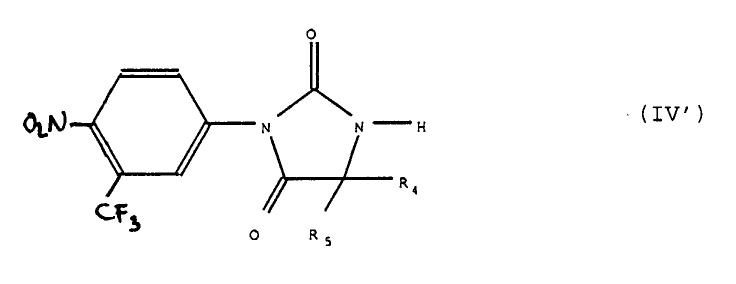 pour obtenir un produit de formule (IV") : 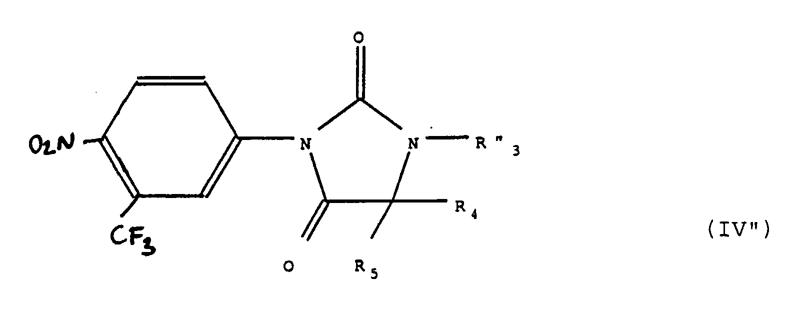 produit de formule (IV") qui représente ou ne représente pas un produit de formule (I) et que, pour obtenir si nécessaire ou si désiré un produit de formule (I), l'on soumet à l'une quelconque ou plusieurs des réactions suivantes dans un ordre quelconque :
   b) réaction de transformation du groupement >C=O en groupements >C=S, les dits produits de formule (I) ainsi obtenus étant sous toutes les formes isomères possibles racémiques, énantiomères et diastéréoisomères.

L'action des produits de formule (II) avec les produits de formule (III) est effectuée de préférence dans un solvant organique tel que le tétrahydrofuranne ou le dichloroéthane 5 mais on peut également utiliser l'éther éthylique ou l'éther isopropylique.

On opère en présence d'une base tertiaire telle que la triéthylamine ou encore la pyridine ou la méthyléthyl-pyridine.

La réaction éventuelle d'hydrolyse du groupement >C=NH en groupement cétone est effectuée de préférence à l'aide d'un acide tel que l'acide chlorhydrique aqueux par exemple au reflux.

Lorsque l'hydrolyse du groupement >C=NH en groupement carbonyle est effectuée sur une molécule comportant également un groupement >C=S, celui-ci peut être transformé en groupement >C=O.

La réaction de transformation du groupement >C=O en groupement >C=S est effectuée à l'aide du réactif dit de Lawesson de formule : 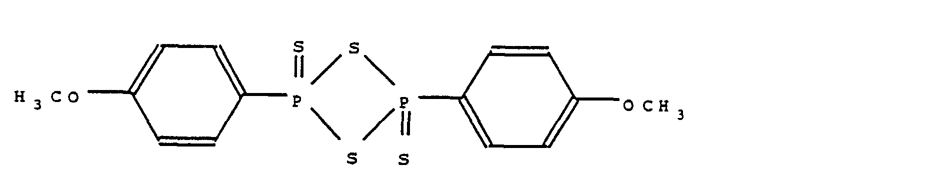 qui est un produit commercialisé par exemple par la firme FLUKA et dont l'utilisation est décrite par exemple dans la publication : Bull. Soc. Chim. Belg. vol 87, N° 3, (1987) p. 229.

Lorsque l'on part d'une molécule comportant deux fonctions >C=O et que l'on veut obtenir un produit ne comportant qu'une seule fonction >C=S, on opère en présence d'un déficit de réactif de Lawesson. On obtient alors en général un mélange de trois produits : chacun des deux produits comportant une fonction >C=O et une fonction >C=S et le produit comportant deux fonctions >C=S. Ces produits peuvent être ensuite séparés par les méthodes usuelles telles que la chromatographie.

L'action sur le produit de formule (IV), (IVa), (IV') ou (VIII) du réactif de formule Hal-R"₃ est effectuée en présence d'une base forte telle que l'hydrure de sodium ou de potassium. On peut opérer par réaction de transfert de phase en présence de sels d'ammonium quaternaires tels que le tert-butyl ammonium.

La réaction du produit de formule (II) telle que définie ci-dessus avec le produit de formule (VII) telle que définie ci-dessus pour donner le produit de formule (VIII) telle que définie ci-dessus, peut être réalisée notamment en présence de chlorure de méthylène à une température d'environ -30°C.

La présente invention a également pour objet un procédé de préparation des produits de formule (I") : 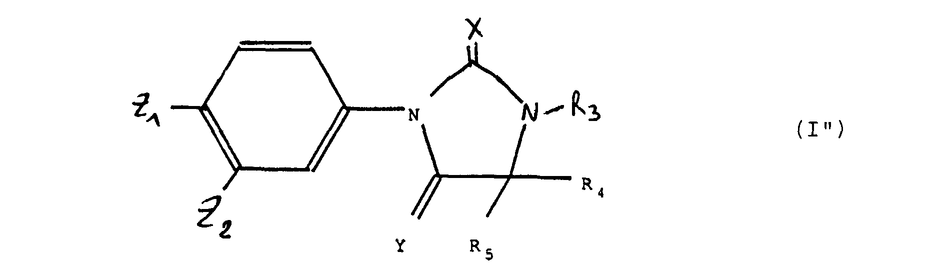 dans laquelle Z₁, Z₂, X, Y, R₃, R₄ et R₅ sont définis comme ci-dessus étant entendu que lorsque -CX-NR₃- représente un groupement -CO-N(R"'₃)- dans lequel R"'₃ représente un atome d'hydrogène ou un radical alkyle linéaire ou ramifié ayant au plus 4 atomes de carbone et Y représente un atome d'oxygène, Z₁ représente un radical cyano, procédé caractérisé en ce que l'on fait réagir un produit de formule (V) : 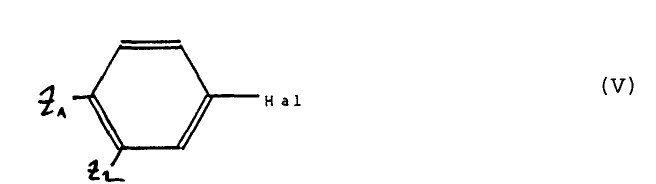 dans laquelle Z₁ et Z₂ ont les significations précédentes et Hal représente un atome d'halogène avec un produit de formule (VI) : 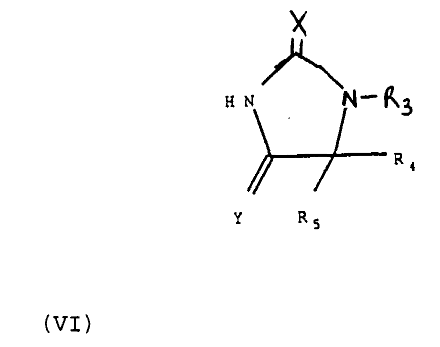 dans laquelle -CX-NR₃-, R₄, R₅ et Y ont la signification indiquée ci-dessus, la réaction s'effectuant en présence d'un catalyseur et éventuellement d'un solvant.

En ce qui concerne les produits de formule (V), le terme Hal désigne de préférence l'atome de chlore, mais peut aussi représenter un atome de brome ou d'iode.

Le rôle du catalyseur est vraisemblablement de piéger l'halogénure d'hydrogène qui se dégage et ainsi de faciliter la réaction de condensation du produit de formule (V) avec le produit de formule (VI) pour donner le produit recherché.

L'invention a plus précisément pour objet un procédé tel que défini ci-dessus dans lequel le catalyseur est un métal sous forme native ou oxydée ou une base.

Quand le catalyseur utilisé est un métal, ce métal peut être du cuivre ou du nickel. Il peut être sous forme native, sous forme d'oxyde métallique ou encore sous forme de sels métalliques.

Les sels métalliques peuvent être un chlorure ou un acétate.

Quand le catalyseur est une base, cette base peut être par exemple la soude ou la potasse et on peut, si désiré, ajouter au milieu réactionnel du diméthylsulfoxyde.

L'invention a plus précisément pour objet un procédé tel que défini ci-dessus dans lequel le catalyseur est choisi parmi l'oxyde cuivreux, l'oxyde cuivrique, le cuivre sous forme native et une base telle que la soude ou la potasse.

Le cuivre sous forme native utilisé comme catalyseur est préférentiellement sous forme de poudre.

L'invention a particulièrement pour objet un procédé tel que défini ci-dessus dans lequel le catalyseur est l'oxyde cuivreux.

Le solvant utilisé est préférentiellement choisi parmi des éthers à haut point d'ébullition tels que, par exemple, l'oxyde de phényle, le diglyme, le triglyme et le diméthylsulfoxyde mais peut être également, par exemple, une huile à haut point d'ébullition telle que la paraffine ou la vaseline.

L'invention a plus particulièrement pour objet un procédé tel que défini ci-dessus caractérisé en ce que l'on opère en présence d'un solvant de type éther tel que l'oxyde de phényle, le diglyme, le triglyme ou le diméthylsulfoxyde.

L'invention a tout particulièrement pour objet un procédé tel que défini ci-dessus dans lequel le solvant utilisé est l'oxyde de phényle ou le triglyme.

Le procédé de préparation du produit recherché défini ci-dessus peut être réalisé sous pression ou à la pression atmosphérique, à une température préférentiellement élevée.

L'invention a ainsi pour objet un procédé tel que défini ci-dessus caractérisé en ce que la réaction est réalisée à une température supérieure à 100°C et de préférence supérieure à 150°C.

L'invention a plus précisément pour objet un procédé tel que défini ci-dessus caractérisé en ce que la réaction est réalisée pendant plus de 2 heures.

L'invention a très précisément pour objet un procédé tel que défini ci-dessus caractérisé en ce que la réaction est réalisée en présence d'oxyde cuivreux, dans le triglyme, à une température supérieure ou égale à 200°C et pendant plus de 3 heures.

Les produits objet de la présente invention possèdent d'intéressantes propriétés pharmacologiques, notamment ils se fixent sur le récepteur des androgènes et ils présentent une activité anti-androgénique.

Des tests donnés dans la partie expérimentale illustrent ces propriétés.

Ces propriétés rendent les produits de formule (I) tels que définis ci-dessus, de la présente invention utilisables comme médicaments principalement pour :
- le traitement des adénomes et des néoplasies de la prostate ainsi que dans l'hypertrophie bénigne de la prostate, seul ou en association avec des analogues de la LHRH. Ils peuvent également être utilisés dans le traitement de tumeurs bénignes ou malignes possédant des récepteurs aux androgènes et plus particulièrement les cancers du sein, de la peau, des ovaires, de la vessie, du système lymphatique, du rein et du foie,
- le traitement d'affections cutanées tel que l'acné, l'hyperséborrhée, l'alopécie ou l'hirsutisme. Ces produits peuvent donc être utilisés en dermatologie seuls ou en association avec des antibiotiques tels que les dérivés des acides azélaique et fusidique, l'érythromycine, ainsi que des dérivés de l'acide rétinoïque ou un inhibiteur de la 5α-réductase tel que le (5α, 17β)-1,1-diméthyléthyl 3-oxo 4-aza-androst-1-ène 17-carboxamide (ou Finastéride, Merck 11ème ed.) pour le traitement de l'acné, l'alopécie ou l'hirsutisme. Ils peuvent également être associés à un produit stimulant la croissance des cheveux tel que le Minoxidil pour le traitement de l'alopécie.

Les produits de formule (I) tels que définis ci-dessus, sous forme radioactive (tritium, carbone 14, iode 125 ou fluor 18) peuvent encore être utilisés comme marqueur spécifique des récepteurs aux androgènes. Ils peuvent aussi être utilisés en diagnostic en imagerie médicale.

Les produits de formule (I) tels que définis ci-dessus, peuvent également être utilisés dans le domaine vétérinaire pour le traitement de troubles comportementaux tel que l'agressivité, d'affection androgénodépendantes, tel que le circum analum chez le chien et de tumeurs présentant des récepteurs aux androgènes. Ils peuvent également être utilisés pour provoquer une castration chimique chez l'animal.

L'invention a donc pour objet l'application, à titre de médicaments, des produits de formule (I) tels que définis ci-dessus, pharmaceutiquement acceptables.

L'invention a également pour objet l'application, à titre de médicaments, des produits suivants :
- 2-(trifluorométhyl) 4-(4-(hydroxyméthyl) 4-méthyl 2,5-dioxo 1-imidazolidinyl) benzonitrile,
- 4-(3,4-diméthyl) 4-(hydroxyméthyl) 5-oxo 2-thioxo 1-imidazolidinyl) 2-(trifluorométhyl) benzonitrile,
- 2-(trifluorométhyl) 4-(4-(hydroxyméthyl) 3,4-diméthyl 2,5-dioxo 1-imidazolidinyl) benzonitrile,
- 4-(2,5-dioxo 3-(2-fluoroéthyl) 4-(hydroxyméthyl) 4-méthyl 1-imidazolidinyl) 2-(trifluorométhyl) benzonitrile,
- 4-(4,4-bis(hydroxyméthyl)-2,5-dioxo-1-imidazolidinyl)-2-(trifluorométhyl) -benzonitrile,
- 4-(4-(fluorométhyl) 3,4-diméthyl) 2,5-dioxo 1-imidazolidinyl) 2-(trifluorométhyl) benzonitrile,
- 4-(3,4-diméthyl) 4-(fluorométhyl) 5-oxo 2-thioxo 1-imidazolidinyl) 2-(trifluorométhyl) benzonitrile,
- 4-(2,5-dioxo 3-(2-fluoroéthyl) 4-(fluorométhyl) 4-méthyl 1-imidazolidinyl) 2-(trifluorométhyl) benzonitrile,
- 1,5-diméthyl 5-(fluorométhyl) 3-(4-nitro 3-(trifluorométhyl) phényl) 2,4-imidazolidinedione,
- 3-(4-cyano 3-(trifluorométhyl) phényl) 2,4-dioxo 5-(fluorométhyl) 5-méthyl 1-imidazolidinacétonitrile,
- 4-(4,4-bis-(fluorométhyl) 3-méthyl 5-oxo 2-thioxo 1-imidazolidinyl 2-(trifluorométhyl) benzonitrile.

Les produits peuvent être administrés par voie parentérale, buccale, perlinguale, rectale ou topique.

L'invention a aussi pour objet les compositions pharmaceutiques, caractérisées en ce qu'elles renferment, à titre de principe actif, un au moins des médicaments de formule (I)tels que définis ci-dessus.

Ces compositions peuvent être présentées sous forme de solutions ou de suspensions injectables, de comprimés, de comprimés enrobés, de capsules, de sirops, de suppositoires, de crèmes, de pommades et de lotions. Ces formes pharmaceutiques sont préparées selon les méthodes usuelles. Le principe actif peut être incorporé à des excipients habituellement employés dans ces compositions, tels que les véhicules aqueux ou non, le talc, la gomme arabique, le lactose, l'amidon, le stéarate de magnésium, le beurre de cacao, les corps gras d'origine animale ou végétale, les dérivés paraffiniques, les glycols, les divers agents mouillants, dispersants ou émulsifiants, les conservateurs.

La dose usuelle, variable selon le sujet traité et l'affection en cause, peut être, par exemple, de 10 mg à 500 mg par jour chez l'homme, par voie orale.

Les produits de formule (II) utilisés au départ de l'invention peuvent être obtenus par action du phosgène lorsque X représente un atome d'oxygène ou du thiophosgène lorsque X représente un atome de soufre sur l'amine correspondante de formule (A) :

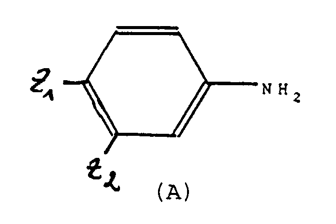

Un exemple d'une telle préparation est donné ci-après dans la partie expérimentale. Un produit de ce type est décrit également dans le brevet français BF 2.329.276.

Les amines de formule (A) sont décrites dans le brevet européen EP 0.002.892 ou le brevet français BF 2.142.804.

Les produits de formule (III) ou (III') sont connus ou peuvent être préparés à partir de la cyanhydrine correspondante selon le procédé décrit dans les publications : J. Am. Chem. Soc. (1953), 75, 4841, BEIL I 4 526 ou J. Org. Chem. 27 2901 (1962).

Les produits de formule (III) dans lesquels R ₃ est différent d'un atome d'hydrogène peuvent être obtenus par action d'un produit de formule R"₃ Hal sur le 2-cyano 2-amino propane dans les conditions énoncées ci-dessus pour l'action de R"₃ Hal sur les produits de formule (IV). Un exemple de préparation de ce type est décrit dans la référence :
- Jilek et Coll. Collect. Czech. Chem. Comm. 54(8) 2248 (1989).

Les produits de formule (IV') sont décrits dans le brevet français BF 2.329.276.

Les produits de formules (V) et (VI), utilisés au départ d'un procédé, objet de l'invention, pour l'obtention des produits de formule (I") tels que définis ci-dessus, sont connus et disponibles dans le commerce ou peuvent être préparés selon des méthodes connues de l'homme de métier.

La préparation de produits de formule (VI) est décrite notamment dans les publications suivantes :
- Zhur. Préklad. Khim. 28, 969-75 (1955) (CA 50, 4881a, 1956)
- Tétrahédron 43, 1753 (1987)
- J. Org. 52, 2407 (1987)
- Zh. Org. Khim. 21, 2006 (1985)
- J. Fluor. Chem. 17, 345 (1981)
ou dans les brevets :
- allemand DRP 637.318 (1935)
- européen EP 0.130.875
- japonais JP 81.121.524.

Les produits de formule (VI) qui sont des dérivés de l'hydantoïne sont largement utilisés et cités dans la littérature comme par exemple dans les articles suivants :
- J. Pharm. Pharmacol., 67, Vol. 19(4), p. 209-16 (1967)
- Khim. Farm. Zh., 67, Vol. 1 (5) p. 51-2
- Brevet allemand 2.217.914
- Brevet européen 0.091.596
- J. Chem. Soc. Perkin. Trans. 1, p. 219-21 (1974).

Les exemples suivants illustrent l'invention sans toutefois la limiter.

### EXEMPLE 1 : 2-(trifluorométhyl) 4-(4-(hydroxyméthyl) 4-méthyl 2,5-dioxo 1-imidazolidinyl) benzonitrile

### Stade A : 1-(tétrahydro 2H-pyran-2-yl) oxy) 2-propanone

On introduit 50 g d'hydroxyacétone, 100 cm³ de chlorure de méthylène et 0,5 g d'acide paratoluène sulfonique monohydraté 1 %. On ajoute ensuite en 5 heures à 20°C, 62,44 g de 3,4-dihydro 2-4-pyran. Après 2 h 30 d'introduction, on ajoute 0,5 g d'acide paratoluène sulfonique puis agite 1 h 30 et ajoute 100 cm³ d'eau saturée de bicarbonate de sodium. On agite 5 mn, à pH alcalin puis décante et extrait au chlorure de méthylène, lave ensuite à l'eau, sèche les phases organiques,filtre et amène à sec. On obtient 101,8 g de produit attendu (huile jaune pâle).

### Stade B : 2-amino 2-méthyl 3-((tétrahydro 2H-pyran 2-yl) oxy) propanenitrile

On agite sous ultasons pendant 1 heure en maintenant la température à 40°C, 77,3 g de cyanure de potassium, 178,1 g d'alumine et 70 g de chlorure d'ammonium dans 1 l d'acétonitrile.

On ajoute alors 95 g du produit obtenu au stade A) ci-dessus, puis rince avec 0,2 l d'acétonitrile et agite pendant environ 21 heures.

On filtre, rince à l'acétonitrile et sèche. On purifie sur silice (éluant cyclohexane-acétate d'éthyle 1-1) et récupère 65,5 g de produit attendu (huile jaune).
Spectre IR : CHCl₃

| | |
|---|---|
| C≡N | 2230 |
| NH | 3393 |
| | 3330 |

### Stade C : 2-(trifluorométhyl) 4-(4-(hydroxyméthyl) 4-méthyl 2,5-dioxo 1-imidazolidinyl) benzonitrile

a) **Condensation 2-(trifluorométhyl) 4-[5-imino 4-méthyl 2-oxo 4-[[(tétrahydro 2-H-pyran 2-yl) oxy] méthyl] 1-imidazolidinyl] benzonitrile**
   On introduit à 20°C ± 2°C, 12,77 g du produit obtenu au stade B ci-dessus, dans 127,7 ml de chlorure de méthylène. Puis en environ 1 h 30 mn, sous agitation à -30°C ± 3°C, on ajoute une solution préalablement filtrée de 11,4 g du produit obtenu à la préparation de l'exemple 7 de la demande de brevet européen EP 0 494 819 dans 171 ml de chlorure de méthylène et agite environ 1 heure à -30°C ± 3° puis évapore le solvant sous pression réduite à 40°C. On obtient 24,7 g du produit de condensation attendu utilisé tel quel pour l'hydrolyse méthanolique.
b) **Hydrolyse** 2-(trifluorométhyl) 4-(4-(hydroxyméthyl) 4-méthyl 2,5-dioxo 1-imidazolidinyl) benzonitrile
   On introduit, sous agitation, à 20° ± 2°, 21,3 g de produit obtenu ci-dessus en a, dans 213 ml de méthanol. Puis on ajoute, en 2 mn, 67 ml d'acide chlorhydrique 2N. On porte au reflux pendant 1 h puis laisse refroidir sous agitation. On concentre en distillant environ 100 ml de méthanol, place le milieu réactionnel sous agitation magnétique pendant environ 1 heure à une température de 0°/+ 5°C puis essore.

On purifie les cristaux obtenus, ajoute 3 volumes de méthanol, porte au reflux 15 mn, puis laisse refroidir sous agitation à 20-25° et essore. On obtient 10,7 g de produit attendu (cristaux blancs). F = 218°C.

| Microanalyse : | | |
|---|---|---|
| | Théorie | Produit séché à 60°C |
| C % | 49,85 | 49,7 |
| N % | 13,4 | 13,4 |
| F % | 18,19 | 18,0 |
| H % | 3,22 | 3,2 |

| | |
|---|---|
| IR Absorption complexe région OH/NH | |
| -C≡N | ~ 2230 cm⁻¹ |
| >=O | 1780 - 1735 cm⁻¹ |
| Aromatiques | 1604 - 1575 - 1503 cm⁻¹ |

### EXEMPLE 2 : 2-(trifluorométhyl) 4-(4-(hydroxyméthyl) 3,4-diméthyl 2,5-dioxo 1-imidazolidinyl) benzonitrile,

1) Formation de l'éther tétrahydropyranique
   On introduit 626 mg du produit de l'exemple 1, 10 ml de tétrahydrofuranne, 30 mg d'acide paratoluène sulfonique, H₂O et 2 ml de dihydropyrane. Au bout d'environ 30 mn, on verse sur 10 ml de bicarbonate de sodium et 1 ml de triéthylamine et extrait au chloroforme. On lave la phase organique à l'eau salée, sèche et évapore le solvant. On purifie sur silice (éluant CH₂Cl₂, MeOH). On obtient 830 mg de l'éther attendu.
2) Méthylation de l'azote
   On introduit 103 mg d'hydrure de sodium à 50 % et ajoute en environ 40 mn, 830 mg de l'éther obtenu ci-dessus en 1) dans 7 ml diméthylformamide, puis 10 mn après la fin de cessation du dégagement d'hydrogène, on place dans un bain d'eau et ajoute 0,18 ml d'iodure de méthyle et 0,5 ml de diméthylformamide. Après 30 mn de réaction, on verse sur 40 ml d'eau contenant environ 0,5 g phosphate monopotassique et extrait à l'éther, puis lave la phase organique à l'eau salée, sèche et évapore le solvant sous pression réduite. On purifie sur silice (éluant CH₂Cl₂-Me₂CO (95-5)). On obtient 770 mg de produit utilisé tel quel pour le stade suivant.
3) Hydrolyse de l'éther tétrahydropyrranique
   On introduit 770 mg de l'éther N-méthylé obtenu ci-dessus en 2), dans 10 ml de méthanol, 1,5 ml d'acide chlorhydrique 2N et chauffe à environ 40°C.

Après 30 mn, on ramène à température ambiante, verse sur 20 ml de bicarbonate de sodium, extrait au chloroforme, lave à l'eau salée, sèche et évapore le solvant sous pression réduite. On purifie sur silice (éluant CH₂Cl₂-Me₂CO (3-1)). On recristallise 111 mg du produit brut obtenu ci-dessus dans 5 ml d'isopropanol à chaud, concentre à environ 1 ml et glace pendant 16 heures. On filtre, sèche et obtient 90 mg du produit attendu (cristaux blancs) F=178-179°C.

| Micro-analyse | | | | |
|---|---|---|---|---|
| | C | H | F | N |
| % calculés | 51,38 | 3,70 | 17,41 | 12,84 |
| % trouvés | 51,5 | 3,7 | 17,5 | 12,8 |

| | |
|---|---|
| IR CHCL₃ | |
| OH | 3620 cm⁻¹ |
| >=O | 1781 - 1728 cm⁻¹ |
| C≡N | 2235 cm⁻¹ |
| Aromatiques | 1615 - 1576 - 1505 cm⁻¹ |

| | | |
|---|---|---|
| UV EtOH | | |
| Max | 262 nm | ε = 13900 |
| Infl | 278 nm | ε = 7200 |
| Infl | 286 nm | ε = 3800 |

### EXEMPLE 3 : 4-(2,5-dioxo 3-(2-fluoroéthyl) 4-(hydroxyméthyl) 4-méthyl 1-imidazolidinyl) 2-(trifluorométhyl) benzonitrile,

a) Alkylation par le 1,2-bromofluoroéthane
   On introduit 104 mg d'hydrure de sodium 5 %, et ajoute goutte à goutte en 30 mn, 830 mg de l'éther obtenu au stade 1) de l'exemple 2 et 7,5 ml de diméthylsulfoxyde. Environ 20 mn après cessation du dégagement d'hydrogène, on ajoute en une fois 0,22 ml de 1,2-bromofluoroéthane. Après environ 1 h de réaction, on verse sur 5 ml d'eau contenant 500 mg de phosphate monopotassique et extrait à l'éther. On lave la phase organique à l'eau puis à l'eau salée, sèche et évapore le solvant sous pression réduite. On purifie sur silice (éluant CH₂Cl₂-Me₂CO (97,5-2,5)) et obtient 743 mg de produit attendu.
b) Hydrolyse de l'éther tétrahydropyrranique
   On introduit 743 mg du produit obtenu ci-dessus en 1) dans 10 ml de méthanol, 1,5 ml de d'acide chlorhydrique 2N et porte à 40°C puis au bout de 45 mn on verse sur 20 ml de bicarbonate de sodium et extrait au chloroforme. On lave la phase organique à l'eau salée, sèche et évapore le solvant sous pression réduite. On purifie sur silice (éluant CH₂Cl₂-Me₂CO (9-1)) puis dissout les cristaux obtenus dans 20 ml d'isopropanol à 60°C, filtre, rince et concentre à environ 5 ml, glace environ 1 h et essore. On obtient 466 mg du produit attendu (cristaux blancs). F = 146-147°C.

| Micro-analyse | | | | |
|---|---|---|---|---|
| | C | H | F | N |
| % calculés | 50,15 | 3,65 | 21,15 | 11,70 |
| % trouvés | 50,1 | 3,50 | 20,9 | 11,7 |

| | |
|---|---|
| IR CHCL₃ | |
| OH | 3612 cm⁻¹ |
| >=O | 1782 (m) - 1727 (f) cm⁻¹ |
| C≡N | 2235 cm⁻¹ |

| | | |
|---|---|---|
| UV EtOH | | |
| Max | 260 nm | ε = 15500 |
| Infl | 278 nm | ε = 6700 |
| Infl | 286 nm | ε = 3300 |

### EXEMPLE 4 : 4-(3,4-diméthyl) 4-(hydroxyméthyl) 5-oxo 2-thioxo 1-imidazolidinyl) 2-(trifluorométhyl) benzonitrile

### Stade A : (±) 2-méthyl 2-(méthylamino) 3-[(tétrahydro 2-H pyran 2-yl) oxy] propanenitrile

On introduit 1,54 g de chlorhydrate de méthylamine en solution dans 10 cm³ d'eai et 3,35 g de cétone obtenu au stade A de l'exemple 1 et agite environ 10 mn la suspension obtenue.

On coule en 10 mn, 1,06 g de NaCN en solution dans 5 cm³ d'eau et agite une nuit à température ambiant.

On extrait au chlorure de méthylène, lave avec une solution saturée de chlorure de sodium, sèche et évapore le solvant sous pression réduite. On obtient 3,72 g de produit attendu (huile jaune), utilisé tel quel au stade suivant.

| | |
|---|---|
| IR CHCL₃ | |
| NH | ~ 3345 cm⁻¹ |
| C≡N | ~ 2230 cm⁻¹ |

### Stade B : 2- (trifluorométhyl) 4-[5-imino 3,4-diméthyl 4-[[(tétrahydro 2-H-pyran 2-yl) oxy] méthyl] 2-thioxo 1-imidazolidinyl] benzonitrile

On introduit 2,38 g d'aminonitrile en solution dans 8 cm³ de 1,2-dichloroéthane et ajoute 0,5 cm³ de triéthylamine, refroidit à une température de -5° à 0°C et coule en 20 mn à une température inférieure à 0°C, 2,75 g de l'isothiocyanate obtenu à la préparation de l'exemple 11 de la demande de brevet européen EP 0 494 819 en solution dans 17 cm³ de 1,2-dichloroéthane. On laisse revenir à température ambiante et maintient l'agitation environ 2 heures, sèche et évapore le solvant sous pression réduite.

Après purification sur silice (éluant CH₂Cl₂-acétone (92-8)), on obtient 3,31 g de produit attendu.

| | |
|---|---|
| IR CHCl₃ | |
| >C=NH | 3308 cm⁻¹ |
| -C≡N | 2236 cm⁻¹ |
| >C=N | 1679 cm⁻¹ |
| >C=S | 1614 cm⁻¹ |
| Aromatique | 1575 cm⁻¹ |
| | 1505 cm⁻¹ |
| | 1496 cm⁻¹ |

### Stade C : 4-(3,4-diméthyl) 4-(hydroxyméthyl) 5-oxo 2-thioxo 1-imidazolidinyl) 2-(trifluorométhyl) benzonitrile

A 3,25 g du produit obtenu au stade B ci-dessus, en solution dans 35 cm³ de méthanol, on ajoute goutte à goutte 19 cm³ d'acide chlorhydrique 2N et porte au reflux pendant 35 mn.

On neutralise avec une solution de bicarbonate de sodium, extrait au chloroforme, lave la phase organique avec une solution saturée de chlorure de sodium, sèche et évapore le solvant sous pression réduit. Après purification sur silice (éluant CH₂Cl₂-ACOEt (85-15), puis recristallisation dans 10 cm³ d'isopropanol, on obtient 1,90 g de produit attendu (cristaux blancs). F = 167°-168°.

| Micro-analyse | | | | | |
|---|---|---|---|---|---|
| | C | H | N | F | S |
| % calculés | 48,98 | 3,52 | 12,24 | 16,60 | 9,34 |
| % trouvés | 48,9 | 3,6 | 12,1 | 16,7 | 9,1 |

| | |
|---|---|
| IR CHCl₃ | |
| Absence de C=NH | |
| -OH | 3620 cm⁻¹ |
| >C=O | 1759 cm⁻¹ |
| >C≡N | 2238 cm⁻¹ |
| Aromatique | 1610 cm⁻¹ |
| + | 1576 cm⁻¹ |
| Syst. conjugué | 1505 cm⁻¹ |
| | 1494 cm⁻¹ |

### EXEMPLE 5 : 1,5-diméthyl 5-(hydroxyméthyl) 3-(4-nitro 3-(trifluorométhyl) phényl) 2,4-imidazolidinedione

### Stade A : Formation de l'éther tétrahydropyranique

On procède comme en 1) de l'exemple 2 ci-dessus, en remplaçant dans cette préparation le produit de l'exemple 1 par 870 mg du produit obtenu comme à l'exemple 2 de la demande de brevet européen EP 0305270 dans 13 ml de tétrahydrofuranne, 40 mg d'acide paratoluène sulfonique, H₂O, 2,6 ml de dihydropyran. Au bout d'environ 15 mn on verse le mélange réactionnel sur 10 ml d'une solution saturée de bicarbonate de sodium et 1 ml de triéthylamine et extrait au chloroforme. On lave la phase organique à l'eau salée, sèche, évapore le solvant sous pression réduite et purifie sur silice (éluant CH₂Cl₂-MeOH (95-5)) et obtient le produit attendu.

### Stade B : Méthylation de l'azote

On procède comme en 2) de l'exemple 2 ci-dessus, à partir du produit obtenu en 1) ci-dessus, et obtient le produit attendu.

### Stade C : Hydrolyse de l'éther tétrahydropyranique.

On procède comme en 3) de l'exemple 2 ci-dessus, à partir de 955 mg du produit obtenu en 2) ci-dessus et obtient 698 mg de produit attendu (cristaux blancs). F = 153-154°C.

| Micro-analyse | | | | |
|---|---|---|---|---|
| | C | H | F | N |
| % calculés | 44,96 | 3,48 | 16,41 | 12,10 |
| % trouvés | 45,0 | 3,50 | 16,3 | 12,1 |

| | |
|---|---|
| IR CHCl₃ | |
| OH | 3620 cm⁻¹ |
| >C=O | 1682 - 1727 cm⁻¹ |
| Aromatique et bande NO₂ | 1618 - 1596 - 1545 - 1498 cm⁻¹ |

| | | |
|---|---|---|
| UV EtOH | | |
| Infl | 214 nm | ε = 13000 |
| Max | 277 nm | ε = 6100 |
| Infl | 320 nm | |

### EXEMPLE 6 : 4-(4-(fluorométhyl) 3,4-diméthyl) 2,5-dioxo 1-imidazolidinyl) 2-(trifluorométhyl) benzonitrile

Dans 1 ml de tétrahydrofuranne, refroidi à environ -60°C on ajoute 0,2 ml de diéthylaminosulfure trifluorure, puis goutte à goutte la solution refroidie à -60°C de 0,2 g du produit de l'exemple 2 et 6,5 ml de tétrahydrofuranne.

On laisse remonter à température ambiante, puis chauffe à 30°C. Après 1 h, on verse sur 18 ml de bicarbonate de sodium et extrait à l'éther. On lave la phase organique à l'eau salée, sèche et évapore les solvants sous pression réduite. On purifie sur silice (éluant CH₂Cl₂-cyclohexane (9-1)) puis on dissout les cristaux obtenus dans 30 ml d'isopropanol à 60°C, filtre, rince avec 2 ml d'isopropanol, concentre à environ 5 ml et glace une nuit. On essore, sèche et obtient 136 mg de produit attendu (cristaux blancs). F = 153-154°C.

| Micro-analyse | | | | |
|---|---|---|---|---|
| | C | H | F | N |
| % calculés | 51,07 | 3,37 | 23,08 | 12,76 |
| % trouvés | 50,8 | 3,2 | 25,0 | 12,7 |

| | |
|---|---|
| IR CHCl₃ | |
| C=N | 2240 cm⁻¹ |
| >C=O | 1785 - 1733 cm⁻¹ |
| Aromatique | 1616 - 1575 - 1505 cm⁻¹ |

| | | |
|---|---|---|
| UV EtOH | | |
| Max | 259 nm | ε = 15200 |
| Infl | 278 nm | ε = 5800 |
| Infl | 286 nm | ε = 2900 |

### EXEMPLE 7 : 4-(3,4-diméthyl) 4-(fluorométhyl) 5-oxo 2-thioxo 1-imidazolidinyl) 2-(trifluorométhyl) benzonitrile

On refroidit 2 cm³ de tétrahydrofuranne anhydre, à -60°C et coule goutte à goutte en environ 15 mn à une température comprise entre -60°C et -53°C, 0,88 cm³ de diéthylaminosulfure trifluorure puis 0,930 g du produit de l'exemple 4 en solution dans 7 cm³ de tétrahydrofuranne anhydre. Après retour à température ambiante, on maintient environ 30 mn à environ 30°C. On verse alors sur 25 cm³ de solution de bicarbonate de sodium + glace. On extrait à l'éther, lave la phase éthérée avec une solution saturée de chlorure de sodium, sèche et évapore les solvants sous pression réduite. Après purification sur silice (éluant CH₂Cl₂-cyclohexane (9-1)) et recristallisation dans l'isopropanol, on obtient après séchage, 1,010 g de produit attendu (cristaux blancs). F = 163°C.

| Micro-analyse | | | | | |
|---|---|---|---|---|---|
| | C | H | F | N | S |
| % calculés | 48,69 | 3,21 | 22,01 | 12,17 | 9,28 |
| % trouvés | 48,6 | 3,10 | 22,2 | 12,1 | 9,5 |

| | |
|---|---|
| IR CHCl₃ | |
| Absence d' OH | |
| N=C | ~ 2238 cm⁻¹ |
| >C=O | 1762 cm⁻¹ |
| Syst. conjugué + | 1615 - 1580 cm⁻¹ |
| Aromatique | 1505 - 1491 cm⁻¹ |

| | | |
|---|---|---|
| UV EtOH | | |
| Max | 235 nm | ε = 19200 |
| Max | 253 nm | ε = 23000 |
| Infl | 265 nm | ε = 18300 |

### EXEMPLE 8 : 4-(2,5-dioxo 3-(2-fluoroéthyl) 4-(fluorométhyl) 4-méthyl 1-imidazolidinyl) 2-(trifluorométhyl) benzonitrile

On refroidit 1 ml de tétrahydrofuranne, à -50°C et ajoute 0,33 ml de diéthylaminosulfure trifluorure puis goutte à goutte une solution préalablement refroidie à environ -50°C de 360 mg du produit de l'exemple 3 et 4 ml de tétrahydrofuranne. On rince avec 0,5 ml de tétrahydrofuranne et laisse remonter à température ambiante puis porte à environ 30°C. On verse alors sur 30 ml de bicarbonate de sodium + 10 g de glace et extrait au chloroforme puis lave la phase organique à l'eau salée et sèche. On purifie sur silice (éluant CH₂Cl₂-acétate d'éthyle (99-1)) puis dissout les cristaux obtenus dans 30 ml d'isopropanol à reflux, filtre, rince avec 1 ml d'isopropanol, concentre à environ 7 ml et laisse reposer 16 heures à 0°C. On essore et obtient après séchage 307 mg de produit attendu (cristaux blancs) F = 137-138°C.

| Micro-analyse | | | | |
|---|---|---|---|---|
| | C | H | F | N |
| % calculés | 49,87 | 3,35 | 26,29 | 11,63 |
| % trouvés | 49,8 | 3,4 | 26,2 | 11,6 |

| | |
|---|---|
| IR CHCl₃ | |
| C≡N | 2235 cm⁻¹ |
| >=O | 1786 - 1730 cm⁻¹ |
| Aromatique | 1616 - 1575 - 1505 cm⁻¹ |

| | | |
|---|---|---|
| UV EtOH | | |
| Max | 258 nm | ε = 16000 |
| Infl | 277 nm | ε = 5300 |
| Infl | 285 nm | ε = 2700 |

### EXEMPLE 11 : 1,5-diméthyl 5-(fluorométhyl) 3-(4-nitro 3-(trifluorométhyl) phényl) 2,4-imidazolidinedione

On introduit 1 ml de tétrahydrofuranne, refroidit à environ -60°C, ajoute 0,09 ml de diéthylaminosulfure trifluorure et ajoute goutte à goutte une solution refroidie à environ -60°C de 210 mg du produit de l'exemple 5 et 2,5 ml de tétrahydrofuranne. On rince avec 0,5 ml de tétrahydrofuranne, laisse remonter à température ambiante, ramène à une température d'environ -60°C et ajoute 0,1 ml de diéthylaminosulfure trifluorure.

Au bout de 1 h 20, on verse sur 8 ml de bicarbonate de sodium et extrait à l'éther. On lave la phase organique à l'eau salée, sèche, évapore le solvant sous pression réduite et purifie sur silice avec pour éluant CH₂Cl₂-Me₂CO (99-1). On obtient 152 mg de produit attendu (cristaux blancs). F = 118-119°C.

| Micro-analyse | | | | |
|---|---|---|---|---|
| | C | H | F | N |
| % calculés | 44,71 | 3,17 | 21,76 | 12,03 |
| % trouvés | 44,9 | 3,1 | 21,42 | 11,9 |

| | |
|---|---|
| IR CHCl₃ | |
| -C=O | 1786 - 1732 cm⁻¹ |
| Aromatique et | 1618 - 1597 - 1546 - 1498 cm⁻¹ |

| | | |
|---|---|---|
| UV EtOH | | |
| Infl | 214 nm | ε = 13400 |
| Max | 267 nm | ε = 6200 |
| Infl | 320 nm | |

### EXEMPLE 16 : 3-(4-cyano 3-(trifluorométhyl) phényl) 2,4-dioxo 5-(hydroxyméthyl) 5-méthyl 1-imidazolidinacétonitrile

On procède comme à l'exemple 2, à partir du produit de l'exemple 1, en remplacant au 2) de l'exemple 2 le iodométhyle par du bromocyanométhyle et obtient ainsi le produit attendu. F = 171°C.

### EXEMPLE 17 : 3-(4-cyano 3-(trifluorométhyl) phényl) 2,4-dioxo 5-(fluorométhyl) 5-méthyl 1-imidazolidinacétonitrile

On procède comme pour la préparation de l'exemple 6, en y remplaçant le produit de l'exemple 2 par le produit de l'exemple 16 et obtient ainsi le produit attendu. F = 175°C.

### EXEMPLE 18 : (±) 4-[2-oxo 5-imino [[(4-fluorophényl) thio] méthyl] 4-méthyl 1-imidazolidinyl 2-trifluorométhyl benzonitrile

### Stade A : (±) 3-[(4-fluorophényl) thiol 2-amino 2-méthyl propane nitrile

A 2,21 g de cyanure de sodium dans 45 ml d'eau, on ajoute successivement une solution de 4,81 g de chlorure d'ammonium dans 12 ml d'eau et 24,6 ml d'ammoniaque 25°Bé puis une solution de 8,32 g de 1-[4-fluorophénylthio] 2-propanone préparé ainsi qu'il est indiqué dans le brevet 82 46399 E, dans 21 ml d'éthanol à 96,2 %. On maintient à 60°C et agite pendant environ 22 h. On refroidit à 0°C/+4°C, rince à l'éthanol, distille, décante, extrait la phase aqueuse au CH₂Cl₂, lave au chlorure de sodium en solution saturée, sèche et évapore le solvant sous pression réduite. On purifie par chromatographie sur silice avec pour éluant cyclohexane-acétate d'éthyle (50-50) et obtient 14,50 g de produit attendu.

| | |
|---|---|
| IR CHCl₃ | |
| Absorption NM | 3382 - 3327 cm⁻¹ |
| Aromatique | 1591 - 1491 cm⁻¹ |
| - C≡N | 2228 cm⁻¹ |

### Stade B : (±) 4-[2-oxo 5-imino [[(4-fluorophényl) thio] méthyl] 4-méthyl 1-imidazolidinyl 2-trilfluorométhyl benzonitrile

On introduit 9,8 g du produit obtenu au stade A ci-dessus, 35 ml de 1,2-dichloroéthane, et ajoute 0,2 ml triéthylamine. On refroidit à 5°C et introduit en 12 mn à une température entre 5°C et 10°C la solution de 7,7 g du produit obtenu à la préparation de l'exemple 7 de la demande de brevet européen EP 0 494 819 et 40 ml de 1,2-dichloroéthane.

On rince avec 5 ml de dichloroéthane et abandonne 16 heures à la température ambiante. On évapore le solvant sous pression réduite, purifie sur silice (éluant CH₂Cl₂-Me₂CO (93-7)), puis dissout dans 100 ml d'isopropanol à environ 60°C, filtre, rince avec 20 ml d'isopropanol chaud, concentre, glace pendant environ 3 h, essore, rince à l'isopropanol glacé et sèche. On obtient 2,815 g de produit attendu (cristaux blancs) F = 150°C.

| Micro-analyse | | | | | |
|---|---|---|---|---|---|
| | C | H | F | N | S |
| % calculés | 54,03 | 3,34 | 17,99 | 13,26 | 7,59 |
| % trouvés | 54,1 | 3,30 | 17,9 | 13,1 | 7,7 |

| | |
|---|---|
| IR CHCl₃ | |
| C=O | 1756 cm⁻¹ |
| C=N | 1669 cm⁻¹ |
| NH | 3444 cm⁻¹ |
| Aromatique | 1614 - 1591 - 1505 - 1491 cm⁻¹ |
| C≡N existe | |

### EXEMPLE 19 : 4-[2,5-dioxo 4-[[(4-fluorophényl) thio] méthyl] 4-méthyl 1-imidazolidinyl] 2-trifluorométhyl benzonitrile

On introduit 3,95 g du produit obtenu à l'exemple 18, 14 ml d'acide chlorhydrique 22°Bé et 14 ml d'eau et chauffe la suspension à reflux.

Au bout d'environ 1 h 30, on ramène à température ambiante, verse sur glace + eau 100 g (1-1) et extrait à l'acétate d'éthyle. On lave la phase organique à l'eau, puis au bicarbonate de sodium en solution saturée, enfin avec du chlorure de sodium en solution saturée et évapore le solvant. On purifie par chromatographie sur silice (éluant CH₂Cl₂-Me₂CO (95-5)). On reprend par 50 ml d'éthanol 100 à 50°C, filtre, rince avec 5 ml d'éthanol chaud, concentre et abandonne 16 heures au réfrigérateur à environ 0° à + 4°C.

On essore, rince à l'éthanol glacé et sèche. On obtient 3,55 g de produit attendu (cristaux blancs). F = 153°C.

| Micro-analyse | | | | | |
|---|---|---|---|---|---|
| | C | H | F | N | S |
| % calculés | 53,9 | 3,09 | 17,95 | 9,92 | 7,57 |
| % trouvés | 53,9 | 3,1 | 18,3 | 9,8 | 7,8 |

| | |
|---|---|
| IR CHCl₃ | |
| C=O | 1791 - 1734 cm⁻¹ |
| NH | 3439 cm⁻¹ |
| C=N | 2236 cm⁻¹ |
| Aromatiques | 1615 - 1591 - 1505 - 1492 cm⁻¹ |

### EXEMPLE 20 : 4-(2,5-dioxo 3-(2-fluoroéthyl) 4-((4-fluorophényl) thiométhyl) 4-méthyl 1-imidazolidinyl 2-(trifluorométhyl) benzonitrile

A 0,031 g de d'hydrure de sodium à 50 % dans l'huile on coule en environ 10 mn goutte à goutte la solution de 0,254 g du produit obtenu comme à l'exemple 19 dans 2,2 cm³ de diméthylsulfoxyde. L'agitation est maintenue environ 40 mn. Puis on ajoute goutte à goutte en environ 5 mn, la solution de 0,54 cm³ de 1-bromo 2-fluoroéthane dans 0,7 cm³ de diméthylsulfoxyde.

Après 30 mn d'agitation, on verse sur 0,4 g de phosphate monosodique, eau + glace. On extrait à l'éther, lave la phase organique avec une solution saturée de chlorure de sodium, sèche et évapore le solvant sous pression réduite. Après purification sur silice (éluant CH₂Cl₂-acétate d'éthyle (100-0,5)) puis recristallisation dans 15 cm³ d'isopropanol, on obtient 0,175 g de produit attendu (cristaux blancs). F = 155°C.

| Micro-analyse | | | | | |
|---|---|---|---|---|---|
| | C | H | F | N | S |
| % calculés | 53,73 | 3,43 | 20,23 | 8,95 | 6,83 |
| % trouvés | 53,5 | 3,2 | 20,5 | 9,0 | 7,1 |

| | |
|---|---|
| IR CHCl₃ | |
| Absence de =C-NH | |
| C≡N | ∼ 2235 cm⁻¹ |
| >C=O | 1780 - 1727 cm⁻¹ |
| Aromatiques | 1616 - 1591 - 1505 - 1492 cm⁻¹ |

| | | |
|---|---|---|
| UV EtOH | | |
| Max | 255 nm | ε = 18600 |
| Infl | 278 nm | ε = 8000 |
| Infl | 287 nm | ε = 4400 |

### EXEMPLE 21 : 4-(4,4-bis(hydroxyméthyl) 3-méthyl 5-oxo 2-thioxo 1-imidazolidinyl) 2-(trifluorométhyl) benzonitrile

### Stade A : 2-(méthylamino) 2-[[(tétrahydro 2-H-pyranl 2-yl) oxy] méthyl] 3-[(tétrahydro 2H-pyran 2-yl) oxy propanenitrile

On introduit 2,7 g de 1,3-bis[(tétrahydro 2H-pyran-2-yl) oxy] 2-propanone obtenu ainsi qu'il est ci-dessous, 5 ml d'eau et 0,77 g de méthylamine chlorhydrate puis ajoute en 5 mn, 503 mg de cyanure de sodium et 3 ml d'eau. Après 2 heures et demie de réaction, on extrait au chlorure de méthylène, lave la phase organique à l'eau salée, évapore le solvant sous pression réduite. On obtient 3,3 g de produit attendu, utilisé tel quel pour le stade suivant.

| | |
|---|---|
| IR CHCl₃ | |
| NH | 3346 cm⁻¹ |
| C≡N | 2230 cm⁻¹ |
| C=O | 1732 cm⁻¹ |

### Préparation du 1,3-bis-[(tétrahydro 2H-pyran 2-yl) oxy] 2-propanone utilisé au départ de l'exemple 21

On chauffe à 70°C, 9 g de 1,3-dihydroxyacétone dimer en suspension dans 60 ml de dioxane et ramène à température ambiante. On ajoute 20 ml de 3,4-dihydro 2,4-pyrane puis 300 mg d'acide paratoluène sulfonique, H₂O en maintenant la température inférieure à 40°C. On maintient 16 heures sous agitation, verse sur 300 ml d'une solution saturée en bicarbonate de sodium, lave la phase organique à l'eau salée, sèche et évapore le solvant sous pression réduite. Après chromatographie du résidu sur silice (éluant cyclohexane-acétate d'éthyle-triéthylamine (8-2-0,5), on obtient 17 g de produit attendu. RF = 0,2.

### Stade B : 2-(trifluorométhyl) 4-[4,4-bis-[[(tétrahydro 2-H-pyran 2-yl) oxy] méthyl] 5-imino 3-méthyl 2-thioxo 1-imidazolidinyl] benzonitrile

On introduit 2,39 g de l'isothiocyanate obtenu à la préparation de l'exemple 11 de la demande de brevet européen EP 0 494 819 et 10 ml de 1,2-dichloroéthane. Puis on ajoute goutte à goutte à la solution refroidie à +5°C, 3,2 g du produit obtenu au stade A ci-dessus, 0,4 ml de triéthylamine, et 10 ml de 1,2-dichloroéthane. Après environ 1 h 20 de chauffage on évapore le solvant et purifie sur silice (éluant acétate d'éthyle 7 cyclo-hexane 3). On obtient 3,82 g du produit attendu.

| | |
|---|---|
| IR CHCl₃ | |
| >C=NH | 3314 cm⁻¹ |
| C≡N | 2230 cm⁻¹ |
| C=N | 1678 - 1670 - 1876 |
| C=S | 1505 - 1495 cm⁻¹ |
| Aromatiques | |

### Stade C : 4-(4,4-bis(hydroxyméthyl) 3-méthyl 5-oxo 2-thioxo 1-imidazolidinyl) 2-(trifluorométhyl) benzonitrile

On introduit 3,8 g du produit obtenu au stade B ci-dessus dans 38 ml de méthanol et 19 ml d'acide chlorhydrique 2N, puis chauffe à reflux. Au bout d'environ 2 h, on verse sur 200 ml d'eau et extrait à l'éther puis à l'acétate d'éthyle. On joint les phases organiques et les lave à l'eau salée puis évapore le solvant. On purifie sur silice (éluant CH₂Cl₂-MeOH (95-5)) puis dissout dans 30 ml d'éther isopropylique au reflux, filtre et concentre partiellement. On glace pendant environ 1 h et essore. On obtient 282 mg de produit attendu (cristaux jaunes). F = 169-170°C.

| Micro-analyse | | | | | |
|---|---|---|---|---|---|
| | C | H | F | N | S |
| % calculés | 46,80 | 3,37 | 15,86 | 11,69 | 8,92 |
| % trouvés | 46,8 | 3,3 | 15,9 | 11,5 | 9,0 |

| | |
|---|---|
| IR Nujol | |
| OH/NH | 3410 - 3385 cm⁻¹ |
| C≡N | 2240 cm⁻¹ |
| C=O | 1720 cm⁻¹ |
| Aromatiques | 1608 - 1580 - 1568 cm⁻¹ |

| | | |
|---|---|---|
| UV EtOH | | |
| Max | 234 nm | ε = 17600 |
| Max | 256 nm | ε = 23200 |

### EXEMPLE 22 : 4-(4,4-bis(1-oxopropoxy) méthyl) 3-méthyl 5-oxo 2-thioxo 1-imidazolidinyl) 2-(trifluorométhyl) benzonitrile

On introduit 200 mg du produit de l'exemple 21 dans 2 ml de pyridine et 25 mg de 4-diméthylamino pyridine puis ajoute 16 ml d'anhydride propionique. Après 25 mn de réaction, on verse sur 20 ml de bicarbonate de sodium et extrait au chlorure de méthylène, lave la phase organique à l'eau salée, sèche, évapore le solvant et distille 3 fois avec 30 ml de toluène. On purifie sur silice (éluant CH₂Cl₂), puis cristallise dans l'éther et obtient 239 mg de produit attendu (cristaux blancs). F = 117-118°C.

| Micro-analyse | | | | | |
|---|---|---|---|---|---|
| | C | H | F | N | S |
| % calculés | 50,95 | 4,27 | 12,09 | 8,91 | 6,80 |
| % trouvés | 51,2 | 4,5 | 12,1 | 8,8 | 6,9 |

| | |
|---|---|
| IR CHCl₃ | |
| >C=O | 1755 - 1762 cm⁻¹ |
| C≡N | 2235 cm⁻¹ |
| Aromatiques et C=S | 1615 - 1580 - 1504 - 1488 cm⁻¹ |

| | | |
|---|---|---|
| UV EtOH | | |
| Infl | 236 nm | ε = 18800 |
| Max | 253 nm | ε = 22600 |
| Infl | 265 nm | ε = 18200 |

### EXEMPLE 23 : 4-(4,4-bis(fluorométhyl) 3-méthyl 5-oxo 2-thioxo 1-imidazolidinyl) 2-(trifluorométhyl) benzonitrile

On refroidit 1 ml de tétrahydrofuranne à -50°C et ajoute 0,66 ml de diéthylaminosulfure trifluorure puis goutte à goutte la solution refroidie à une température d'environ -50°C de 360 mg du produit de l'exemple 21 dans 4 ml de tétrahydrofuranne. On chauffe à une température d'environ 30°C.

Au bout d'environ 30 mn, on verse goutte à goutte sur 50 ml d'une solution aqueuse saturée de bicarbonate de sodium extrait au chloroforme, lave la phase organique à l'eau salée et évapore le solvant. On purifie sur silice avec pour éluant CH₂Cl₂-cyclohexane (9-1) puis dissout dans 20 ml d'isopropanol à une température d'environ 60°C. On filtre, rince avec 1 ml d'isopropanol et concentre, glace 1 h et essore. On obtient 314 mg de produit attendu (cristaux blancs). F = 122-123°C.

| Micro-analyse | | | | | |
|---|---|---|---|---|---|
| | C | H | F | N | S |
| % calculés | 46,28 | 2,77 | 26,15 | 11,56 | 8,82 |
| % trouvés | 46,3 | 2,7 | 25,7 | 11,25 | 8,8 |

| | |
|---|---|
| IR CHCl₃ | |
| C≡N | 2236 cm⁻¹ |
| C=O | 1763 cm⁻¹ |
| Aromatiques | 1615 - 1580 - 1504 - 1487 cm⁻¹ |

| | | |
|---|---|---|
| UV EtOH | | |
| Infl | 237 nm | ε = 20300 |
| Max | 250 nm | ε = 22000 |
| Infl | 266 nm | ε = 17100 |

### EXEMPLE 24 : 4-(4,4-bis(hydroxyméthyl) 3-méthyl 2,5-dioxo 1imidazolidinyl) 2-(trifluorométhyl) benzonitrile

### 1) Formation des éthers tétrahydropyraniques

On introduit 360 mg du produit de l'exemple 21 dans 5 ml de tétrahydrofuranne, 1 ml de 3,4-dihydro 2H pyran, et 15 mg d'acide para toluène sulfonique, H₂O.

Après environ 40 mn, on verse 10 ml d'une solution aqueuse saturée de bicarbonate de sodium et 1 ml triéthylamine, extrait au chloroforme, lave à l'eau salée, sèche et évapore le solvant.

On purifie sur silice (éluant CH₂Cl₂-MeOH (93-7)) et obtient 600 mg de produit attendu, utilisé tel quel pour le stade suivant.

### 2) Passage à l'hydantoïne

On introduit 600 mg du diéther obtenu en 1) dans 4 ml de diméthylformamide et ajoute 55 mg d'hydrure de sodium à 50 %, puis après cessation de dégagement d'hydrogène, 0,09 ml d'iodure de méthyle. Environ 40 mn après, on ajoute successivement 110 mg d'hydrure de sodium à 50 %, puis 10 mn après 0,18 ml d'iodure de méthyle. On verse le mélange réactionnel sur 10 ml d'eau glacée contenant 1,3 g de phosphate monopotassique et extrait à l'éther. On lave la phase organique à l'eau salée, sèche et évapore le solvant. On purifie sur silice (éluant CH₂Cl₂-acétate d'éthyle (92,5-7,5)) et obtient 370 mg de produit attendu, utilisé tel quel pour le stade suivant.

### 3) Déprotection des éthers pyraniques

On introduit 370 mg de produit obtenu ci-dessus en 2) dans 4 ml de méthanol et 2 ml d'acide chlorhydrique 2N puis porte la solution à 60°C pendant environ 2 h.

On verse alors sur 15 ml d'eau salée, sèche, évapore le solvant puis dissout le résidu dans 20 ml d'acétone et évapore à sec. On purifie sur silice (éluant CH₂Cl₂-MeOH (92,5-7,5)) puis recristallise dans l'acétone et obtient 197 mg de produit attendu (cristaux blancs) F = 217-218°C.

| | | |
|---|---|---|
| UV EtOH | | |
| Max | 263 nm | ε = 14600 |
| Infl | 237, 278, 287 nm | |

### EXEMPLE 25 : 4-(4,4-bis(hydroxyméthyl) 3-méthyl 5-imino 2-thioxo 1-imidazolidinyl) 2-(trifluorométhyl) benzonitrile

On procède comme à l'exemple 21 et obtient au stade C de la préparation de l'exemple 21, 421 mg de produit attendu.

| | |
|---|---|
| IR | |
| C≡N | 2230 cm⁻¹ |
| C=N, C=S | 1680 - 1614 - 1580 - 1510 cm⁻¹ |
| Aromatiques | |

### EXEMPLE 26 : 4-(4,4-bis(hydroxyméthyl)-2,5-dioxo-1-imidazolidinyl)-2-(trifluorométhyl)-benzonitrile

### STADE 1 : 1,3 bis [(tétrahydro 2H pyran-2 yl)oxy]2 propanone

On introduit 9 g de 2,5-dihydroxy 1,4-dioxane 2,5-diméthanol dans 60 ml de dioxanne et porte la suspension à environ 70°C pendant 15 minutes puis ramène à la température ambiante. On ajoute alors 20 ml de 3,4-dihydro 2H-pyran et 300 mg d'acide paratoluène sulfonique monohydraté et maintient la température à environ 40°C puis laisse 16 heures à température ambiante.

On verse alors sur un mélange de 300 ml de bicarbonate de sodium solution saturée + 10 ml de triéthylamine et extrait au chlorure de méthylène. On lave la phase organique à l'eau salée, sèche et évapore le solvant.

Après chromatographie sur silice (éluant : cycloacétate d'éthyle/triéthylamine 8/2), on obtient 17 g de produit attendu (sirop jaune pâle).

| ANALYSES : | |
|---|---|
| IR CHCl3 (cm⁻¹) | |
| Absence | OH |
| O=C | 1736 |

### STADE 2 : 2-amino 3-((tétrahydro-2H-pyran-2-yl) oxy) 2-(((tétrahydro-2H-pyran-2-yl) oxy) méthyl) propanenitrile

On introduit 5,6 g du produit obtenu au stade 1 ci-dessus dans 8 ml d'ammoniaque, amène à environ -5°C et ajoute successivement 1,58 g de chlorure d'ammonium et 1,23 g de cyanure de sodium et laisse remonter à température ambiante environ 40 minutes puis chauffe à 40°C ± 5°C sous agitation pendant 16 heures. On ramène à température ambiante et extrait au chloroforme, lave la phase organique à l'eau salée, sèche et évapore le solvant.

Après chromatographie sur silice (éluant : cycloacétate d'éthyle/triéthylamine 3/7), on obtient 4,41 g de produit attendu (sirop jaune pâle).

| ANALYSES : | |
|---|---|
| IR CHCl3 (cm⁻¹) | |
| -CN | 2235 |
| NH2 | 3390-3317 |

### STADE 3 : 4-(5-imino-2-oxo-4,4-bis(((tétrahydro-2H-pyran-2-yl) oxy) méthyl) 1-imidazolidinyl) 2-(trifluorométhyl) benzonitrile

On introduit 570 mg du produit obtenu au stade 2 ci-dessus dans 5 ml d'éther isopropylique et 0,28 ml de triéthylamine et amène à -30°C puis ajoute en une heure 2,32 g d'une solution de produit obtenu à la préparation de l'exemple 7 de la demande de brevet européen EP 0 494 819 à 18,4% dans le 1,2-dichloroéthane.

On ajoute 4 ml de chlorure de méthylène puis laisse remonter à température ambiante, laisse environ 2 heures et évapore le solvant. Après purification sur silice (éluant : chlorure de méthylène/acétone 9/1), on obtient 700 mg de produit attendu.

| ANALYSES : | |
|---|---|
| IR CHCl3 (cm⁻¹) | |
| NH | 3442-3317 |
| -CN | 2235 |
| C=O | 1757 |
| C=N | 1670 |
| Aromatique | 1614-1575-1505 |

### STADE 4 : 4-(4,4-bis(hydroxyméthyl)-2,5-dioxo-1-imidazolidinyl)-2-(trifluorométhyl)-benzonitrile

On introduit 300 mg du produit obtenu au stade 3 ci-dessus dans 3 ml de méthanol et 1,5 ml d'acide chlorhydrique 2N et porte à reflux 1h30.

On ramène à température ambiante, verse sur 5 ml de solution aqueuse de bicarbonate de sodium, extrait à l'acétate d'éthyle puis lave au chlorure de sodium en solution aqueuse saturée, sèche et évapore le solvant.

On ajoute 5 ml de méthanol et purifie sur silice (éluant : chlorure de méthylène-méthanol 9/1).

On reprend dans 20 ml d'isopropanol à reflux puis concentre et obtient 225 mg de produit attendu (cristaux blancs) F : 207-208°C.

| ANALYSES : | |
|---|---|
| IR NUJOL | |
| OH/NH | 3525-3365-3250 cm⁻¹ |
| CN | 2240 cm⁻¹ |
| C=O | 1778-1738 cm⁻¹ |
| Aromatique | 1618-1578-1506 cm⁻¹ |

### EXEMPLE 27 : 4-(4,4-bis(fluorométhyl) 2,5-dioxo 3-méthyl 1-imidazolidinyl) 2-(trifluorométhyl) benzonitrile

On opère comme indiqué à l'exemple 23 en utilisant 120 mg de produit obtenu à l'exemple 24. Après chromatographie sur silice (éluant : CH₂Cl₂-acétate d'éthyle 99-1), on obtient 111 mg de produit attendu. F = 137-138°C.

| ANALYSES : | |
|---|---|
| IR CHCl₃ | |
| C≡N | 2235 cm⁻¹ |
| C=O | 1790-1735 cm⁻¹ |
| Aromatique | 1617-1580-1505 cm⁻¹ |

### EXEMPLE 28 : 4-(2,5-dioxo 3-éthyl 4-(hydroxyméthyl 4-méthyl 1-imidazolidinyl) 2-(trifluorométhyl) benzonitrile

On opère comme indiqué à l'exemple 2, stades 2 et 3, en utilisant 1,033 g d'éther tétrahydropyranique préparé au stade 1 et 0,24 ml d'iodure d'éthyle. On obtient après chromatographie sur silice (éluant : chlorure de méthylène-acétate d'éthyle) 0,796 g de produit attendu que l'on recristallise dans l'isopropanol. F = 138°C.

| ANALYSES : | |
|---|---|
| IR CHCl₃ | |
| OH | 3616 cm⁻¹ |
| C≡N | 2236 cm⁻¹ |
| C=O | 1779 (m)-1725 (F) cm⁻¹ |
| Aromatique | 1617-1506 cm⁻¹ |

### EXEMPLE 29 : 3-(2,5-dioxo 3-éthyl 4-méthyl 4-(2-méthyl 1-oxopropoxy) méthyl 1-imidazolidinyl) 2-(trifluorométhyl) benzonitrile

On opère comme à l'exemple 22 en utilisant 280 mg de produit obtenu à l'exemple 28, 2,5 ml de pyridine, 23 mg de diméthylaminopyridine et 0,16 ml d'anhydride isobutyrique. Après extraction à l'éther, élimination des solvants et chromatographie sur silice (éluant : chlorure de méthylène-acétate d'éthyle 100-1), on obtient 321 mg de produit attendu. F = 85°C.

| ANALYSES : | |
|---|---|
| IR CHCl₃ | |
| C≡N | 2235 cm⁻¹ |
| C=O | 1781 (m)-1728 (F) cm⁻¹ |
| Aromatique | 1615-1575-1505 cm⁻¹ |

### EXEMPLE 30 : Carbonate de (1-(4-cyano 3-(trifluorométhyl) phényl) 2,5-dioxo 3-éthyl 4-méthyl 4-imidazolidinyl) méthyle et de 1-méthyléthyle

On opère comme à l'exemple 22 en utilisant 376 mg de produit obtenu à l'exemple 28, 3,8 ml de pyridine, 25 mg de diméthylaminopyridine, ajoute à 0°C 2,2 ml d'une solution toluénique de chloroformate d'isopropyle (1M/l). Après 30 minutes d'agitation à 0°C puis 3 heures à température ambiante, on ajoute 0,4 ml de la solution de chloroformate d'isopropyle et poursuit l'agitation à température ambiante pendant 2 heures et demie. On verse sur 20 g d'eau glacée, extrait à l'éther, lave la solution organique avec de l'eau salée, sèche et élimine les solvants sous pression réduite. On reprend au toluène, concentre à sec, laisse cristalliser l'huile formée et obtient 422 mg de produit brut que l'on chromatographie sur silice (éluant : chlorure de méthylène-acétate d'éthyle 100-2). On obtient 270 mg de produit attendu. F = 123°C.

| ANALYSES : | |
|---|---|
| IR CHCl₃ | |
| C≡N | 2235 cm⁻¹ |
| C=O | 1782-1744-1729 cm⁻¹ |
| Aromatique | 1616-1578-1505 cm⁻¹ |

### EXEMPLE 31 : 4-(4,4-bis(hydroxyméthyl) 2,5-dioxo 3-(4-hydroxybutyl) 1-imidazolidinyl) 2-trifluorométhyl) benzonitrile.

Composé ne faisant pas partie de l'invention.

### 1) Formation des éthers tétrahydropyraniques.

On opère comme à l'exemple 24 stade 1, en utilisant 331 mg de produit obtenu à l'exemple 26. On extrait au chlorure de méthylène, lave à l'eau salée, sèche, évapore le solvant et obtient, après chromatographie sur silice (éluant : CH₂Cl₂-MeOH 9-1), 500 mg de produit attendu utilisé tel quel pour le stade suivant.

### 2) Hydroxyalkylation.

A 52 mg d'hydrure de sodium, on ajoute goutte à goutte en 20 minutes 456 mg du diéther obtenu ci-dessus dans 3 ml de diméthylsulfoxyde puis 20 minutes après cessation du dégagement d'hydrogène, on ajoute 374 mg de triméthylsilyl-4-iodobutanol. Après 40 minutes de réaction, on verse dans 20 ml d'eau, extrait à l'éther, lave la phase organique à l'eau salée, sèche et évapore le solvant. On obtient 650 mg de produit brut que l'on utilise tel quel pour le stade suivant.

### 3) Hydrolyse des groupements protecteurs.

On introduit 650 mg de produit obtenu ci-dessus dans 7 ml de méthanol et 3 ml d'acide chlorhydrique 2N puis porte la solution à 40°C pendant environ 40 minutes. On verse sur 20 ml de solution aqueuse de bicarbonate de sodium, extrait à l'acétate d'éthyle, lave à l'eau salée, sèche, évapore le solvant. On purifie sur silice (éluant : CH₂Cl₂-MeOH 9-1) et obtient 950 mg de produit attendu.

| ANALYSES : | |
|---|---|
| UV EtOH | |
| Max. 237 nm | ∈ = 8600 |
| Max. 263 nm | ∈ = 14000 |
| Infl. 278 nm | ∈ = 8400 |
| Infl. 287 nm | ∈ = 4200 |

### EXEMPLE 32 : 4-(4,4-bis(hydroxyméthyl) 2,5-dioxo 3-(2-fluoroéthyl) 1-imidazolidinyl) 2-trifluorométhyl) benzonitrile.

### 1) Fluoroalkylation.

On opère comme à l'exemple 3 stade a, en utilisant au départ 5 g du diéther tétrahydropyranique préparé comme indiqué à l'exemple 31 stade 1, et 1,1 ml de 2-bromo 1-fluoroéthane. On obtient 5,31 g de produit attendu.

### 2) Hydrolyse de l'éther tétrahydropyranique.

On opère comme à l'exemple 3 stade b en utilisant au départ 550 mg de produit obtenu ci-dessus, 6 ml de méthanol et 2 ml d'acide chlorhydrique 2N. On obtient, après chromatographie sur silice (éluant CH₂Cl₂-Me₂CO 8-2), 351 mg de produit attendu. F = 138-139°C.

| ANALYSES : | |
|---|---|
| IR NUJOL | |
| OH/NH | 3580-3505 cm⁻¹ |
| C≡N | 2245 cm⁻¹ |
| C=O | 1778-1716 cm⁻¹ |
| Aromatique | 1616-1580-1512 cm⁻¹ |

| | |
|---|---|
| UV EtOH | |
| Max. 260 nm | ∈ = 15300 |
| Infl. 280 nm | ∈ = 3400 |

### EXEMPLE 33 : 4-(4,4-bis(fluorométhyl) 2,5-dioxo 3-(2-fluoroéthyl) 1-imidazolidinyl) 2-trifluorométhyl) benzonitrile.

On refroidit à -50°C sous atmosphère inerte 1 ml de tétrahydrofuranne et ajoute goutte à goutte 0,66 ml de diéthylamino sulfure trifluorure, puis en 5 minutes 375 mg de produit obtenu à l'exemple 32 dans 4 ml de tétrahydrofuranne. On laisse revenir à température ambiante, maintient sous agitation pendant 1 heure, verse dans une solution aqueuse glacée de bicarbonate de sodium, extrait au chloroforme, lave la phase organique à l'eau salée, sèche, évapore le solvant, chromatographie le résidu sur silice (éluant : CH₂Cl₂-cyclohexane 9-1) et obtient 337 mg de produit attendu. F = 136-137°C.

| ANALYSES : | |
|---|---|
| IR CHCl₃ | |
| C≡N | 2235 cm⁻¹ |
| C=O | 1787-1736 cm⁻¹ |
| Aromatique | 1617-1577-1505 cm⁻¹ |

### EXEMPLE 34 : 4-(4,4-bis(2-méthyl 1-oxopropoxy) méthyl) 2,5-dioxo 3-(2-fluoroéthyl) 1-imidazolidinyl) 2-trifluorométhyl) benzonitrile.

On ajoute 0,5 ml d'anhydride isobutyrique à une solution sous atmosphère inerte, comprenant 375 mg de produit obtenu à l'exemple 32, 4 ml de pyridine et 122 mg de diméthylaminopyridine. On agite 30 minutes, verse dans 20 ml de solution aqueuse de bicarbonate de sodium à 50%, sèche et évapore le solvant sous pression réduite. On chromatographie le résidu sur silice (éluant : CH₂Cl₂-AcOEt 95-5) et obtient 457 mg de produit attendu. F = 71-72°C.

| ANALYSES : | |
|---|---|
| IR CHCl₃ | |
| C≡N | 2236 cm⁻¹ |
| C=O | 1789-1733 cm⁻¹ |
| Aromatique | 1616-1505 cm⁻¹ |

| | |
|---|---|
| UV EtOH | |
| Max. 257 nm | ∈ = 17000 |
| Infl. 285 nm | ∈ = 2600 |

### EXEMPLE 35: Carbonate de bis (1-méthyléthyle) et de (3-(4-cyano 3-(trifluorométhyl) phényl) 2,4-dioxo 1-(2-fluoroéthyl) 5-imidazolidinyl) bis (méthylène) et ± racémique de carbonate de (3-(4-cyano 3-(trifluorométhyl) phényl) 2,4-dioxo 1-(2-fluoroéthyl) 5-(hydroxyméthyl) 5-imidazolidinyl) méthyle et de 1-méthyléthyle

On refroidit à -4°C sous atmosphère d'argon, 375 mg de produit obtenu à l'exemple 32 dans 4 ml de pyridine et 122 mg de 4-diméthylaminopyridine. On ajoute goutte à goutte, à -4°C, 550 mg de chloroformiate d'isopropyle. On laisse revenir à température ambiante, poursuit l'agitation pendant 2 heures. La réaction étant incomplète, on ajoute 122 mg de diméthylaminopyridine et 2 ml de chloroformiate d'isopropyle et chauffe 18 heures à 50°C. On ramène à température ambiante, verse dans l'eau salée, extrait à l'acétate d'éthyle, sèche, élimine les solvants, et obtient 570 mg de produit brut que l'on purifie par chromatographie sur silice (éluant : CH₂Cl₂-AcOEt 95-5), pour obtenir 275 mg de dicarbonate (F = 122-123°C) puis (éluant : CH₂Cl₂-Me₂CO 9-1), pour obtenir 156 mg de monocarbonate (F = 154-155°C).

| ANALYSES : | |
|---|---|
| . Dicarbonate | |
| IR CHCl₃ | |
| C≡N | 2238 cm⁻¹ |
| C=O | 1789-1749-1734 cm⁻¹ |
| Aromatique | 1615-1578-1505 cm⁻¹ |

| | |
|---|---|
| UV EtOH | |
| Max. 256 nm | ∈= 15400 |
| Infl. 285 nm | ∈ = 2500 |

| | |
|---|---|
| . Monocarbonate | |
| IR (Nujol) | |
| OH/NH | 3450 cm⁻¹ |
| C≡N | 2250 cm⁻¹ |
| C=O | 1789-1736 cm⁻¹ |
| Aromatique | 1616-1576-1506 cm⁻¹ |

### EXEMPLE 36 : Carbonate de bis (2-méthylpropyle) et de (3-(4-cyano 3-(trifluorométhyl) phényl) 2,4-dioxo 1-(2-fluoroéthyl) 5-imidazolidinyl) bis (méthylène)

On refroidit à -4°C sous atmosphère d'argon, 375 mg de produit obtenu à l'exemple 32 dans 4 ml de pyridine et 122 mg de 4-diméthylaminopyridine. On ajoute goutte à goutte, à -4°C, 550 mg de chloroformiate d'isobutyle. On laisse revenir à température ambiante. Après 40 minutes, on verse le milieu réactionnel dans l'eau, lave à l'eau salée, sèche et élimine les solvants, chromatographie le résidu sur silice (éluant : CH₂Cl₂-AcOEt 92,5-7,5) et obtient 476 mg de produit attendu. F = 109-110°C.

| ANALYSES : | |
|---|---|
| IR CHCl₃ | |
| C≡N | 2236 cm⁻¹ |
| C=O | 1790-1754-1734 cm⁻¹ |
| Aromatique | 1615-1578-1505 cm⁻¹ |

| | |
|---|---|
| UV EtOH | |
| Max. 256 nm | ∈ = 15500 |
| Infl. 285 nm | ∈ = 2700 |

### EXEMPLE 37 :

On a préparé des comprimés ayant la composition suivante:
- Produit de l'exemple 3 100 mg
- Excipient q.s. pour un comprimé terminé à 300 mg
   (Détail de l'excipient : lactose, amidon, talc, stéarate de magnésium).

### EXEMPLE 38 :

On a préparé des comprimés ayant la composition suivante:
- Produit de l'exemple 26 100 mg
- Excipient q.s. pour un comprimé terminé à 300 mg
   (Détail de l'excipient : lactose, amidon, talc, stéarate de magnésium).

### ETUDE PHARMACOLOGIOUE DES PRODUITS DE L'INVENTION

### 1) Etude de l'affinité des produits de l'invention pour le récepteur androgène

Des rats mâles Sprague Dawley EOPS de 180-200 g, castrés de 24 heures, sont sacrifiés, les prostates prélevées, pesées et homogénéisées à 0°C à l'aide d'un potter verre-verre, dans une solution tamponnée (Tris 10mM, saccharose 0,25M, PMSF (phénylméthanesulfonylfluoride) 0,1mM, Molybdate de sodium 20mM, HCl pH 7,4 ; auxquels on ajoute extemporanément 2mM de DTT (DL dithiothreitol), à raison de 1 g de tissu pour 8 ml de tampon.

L'homogénat est ensuite ultracentrifugé à 0°C, 30 minutes à 209 000 g. Des aliquotes du surnageant obtenu (=cytosol), sont incubées 30 minutes et 24 heures à 0°C, avec une concentration constante (T) de Testostérone tritiée et en présence de concentrations croissantes (0 à 2500.10⁻⁹M), soit de testostérone froide, soit des produits à tester. La concentration de Testostérone tritiée liée (B) est ensuite mesurée dans chaque incubat par la méthode d'adsorption au charbon-dextran.

### Calcul de l'affinité relative de liaison (ARL).

On trace les 2 courbes suivantes : le pourcentage de l'hormone tritiée liée B/T en fonction du logarithme de la concentration de l'hormone de référence froide et B/T en fonction du logarithme de la concentration du produit froid testé. On détermine la droite d'équation I₅₀=(B/Tmax + B/Tmin)/2.
B/T max= % de l'hormone tritiée liée pour une incubation de cette hormone tritiée à la concentration (T).
B/T min= % de l'hormone tritiée liée pour une incubation de cette hormone tritiée à la concentration (T) en présence d'un grand excès d'hormone froide (2500.10⁻⁹M).

Les intersections de la droite I₅₀ et des courbes, permettent d'évaluer les concentrations de l'hormone de référence froide (CH) et du produit froid testé (CX) qui inhibent de 50 % la liaison de l'hormone tritiée sur le récepteur. L'affinité relative de liaison (ARL) du produit testé est déterminé par l'équation ARL=100 (CH)/(CX).

On obtient les résultats suivants exprimés en ARL.

### Produit de référence (Testostérone) : 100

| Produit des exemples | Incubation : 24 heures |
|---|---|
| 3 | 6 |
| 6 | 16 |
| 26 | 4 |

### 2) Détermination de l'activité androgène ou anti-androgène des produits de l'invention à l'aide du dosage de l'ornithine décarboxylase (ODC).

- **Protocole de traitement**
Des souris mâles SWISS âgées de 6 semaines, et castrées de 24 heures, reçoivent par voie orale ou percutanée les produits à étudier (suspension en méthyl cellulose à 0,5 % ou en solution dans l'éthanol), simultanément avec une injection sous-cutanée de Propionate de testostérone 3 mg/kg (solution dans l'huile de maïs) pour déterminer l'activité anti-androgène. L'activité agoniste est déterminée en l'absence de propionate de testostérone.
Le Propionate de testostérone est administré sous un volume de 10 ml/kg.
20 heures après les traitements, les animaux sont sacrifiés, les reins prélevés, puis homogénéisés à 0°C , à l'aide d'un broyeur téflon-verre dans 10 volumes de tampon Tris-HCl 50 mM (pH 7,4) contenant 250 uM de phosphate de pyridoxal, 0,1 mM EDTA, et 5 mM de dithiothreitol. L'homogenat est ensuite centrifugé à 209000 g pendant 30 mn.
- **Principe de dosage**
A 37°C, l'ornithine décarboxylase rénale transforme un mélange isotopique d'ornithine froide et d'ornithine tritiée en putrescine froide et putrescine tritiée.
La putrescine est ensuite recueillie sur des papiers sélectifs, échangeurs d'ions. Après séchage, l'excès d'ornithine tritiée et froide non transformée est éliminé, par 3 lavages d'ammoniaque 0,1 M. Les papiers sont séchés, puis la radioactivité est comptée après addition de scintillant Aqualite.
Les résultats sont exprimés en fmoles (10⁻¹⁵ M) de putrescine tritiée formée/heure/mg de protéines.
Les résultats sont exprimés en % d'inhibition de l'ODC des témoins ne recevant que le propionate de testostérone. **Test :** les produits sont administrés par voie percutanée à 1,5 mg/kg sous un volume de 10 µl.

| Produits des exemples | Test |
|---|---|
| 3 | 47 |
| 6 | 84 |

**Conclusion :** Les tests indiqués ci-dessus montrant que les produits de l'invention testée possèdent une forte activité anti-androgène.

## Revendications

1. Les produits de formule générale (I) : 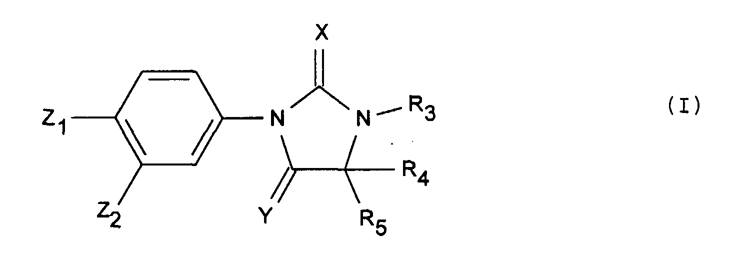 dans laquelle :
Y représente un atome d'oxygène ou un radical NH, Z₂ représente un radical trifluorométhyle et Z₁ représente un radical cyano ou nitro,
X représente un atome d'oxygène ou de soufre,
R₃ représente un atome d'hydrogène ou un radical alkyle ayant au plus 4 atomes de carbone, éventuellement substitué par un ou plusieurs radicaux choisis parmi les atomes d'halogène ou le radical cyano,
R₄ et R₅ sont tels que :
soit l'un représente méthyle et l'autre est choisi parmi les valeurs de R₄ et R₅ ci-aprés,
soit R₄ et R₅ identiques ou différents représentent un radical alkyle linéaire ou ramifié renfermant au plus 4 atomes de carbone substitué par un radical hydroxyle éventuellement substitué par un radical phényle, alkyle renfermant au plus 4 atomes de carbone ou acyle renfermant au plus 7 atomes de carbone ; un atome d'halogène ; ou un radical phénylthio éventuellement substitué par un atome d'halogène ou un radical hydroxyle,
étant entendu que R4 et R5 ne représentent pas l'un méthyle et l'autre hydroxyméthyle ou tertiobutyloxymethyle lorsque Z1 représente nitro, Z2 représente CF3, R3 représente hydrogène, X représente oxygène et Y représente oxygène ou NH,
lesdits produits de formule (I) étant sous toutes les formes isomères racémiques, énantiomères et diastéréoisomères possibles, ainsi que les sels d'addition avec les acides minéraux et organiques ou avec les bases minérales et organiques desdits produits de formule (I).

2. Les produits de formule (I) telle que définie à la revendication 1, dont les noms suivent :
- 2-(trifluorométhyl) 4-(4-(hydroxyméthyl) 4-méthyl 2,5-dioxo 1-imidazolidinyl) benzonitrile,
- 4-(3,4-diméthyl) 4-(hydroxyméthyl) 5-oxo 2-thioxo 1-imidazolidinyl) 2-(trifluorométhyl) benzonitrile,
- 2-(trifluorométhyl) 4-(4-(hydroxyméthyl) 3,4-diméthyl 2,5-dioxo 1-imidazolidinyl) benzonitrile,
- 4-(2,5-dioxo 3-(2-fluoroéthyl) 4-(hydroxyméthyl) 4-méthyl 1-imidazolidinyl) 2-(trifluorométhyl) benzonitrile,
- 4-(4,4-bis(hydroxyméthyl)-2,5-dioxo-1-imidazolidinyl)-2-(trifluorométhyl)-benzonitrile,
- 4-(4-(fluorométhyl) 3,4-diméthyl) 2,5-dioxo 1-imidazolidinyl) 2-(trifluorométhyl) benzonitrile,
- 4-(3,4-diméthyl) 4-(fluorométhyl) 5-oxo 2-thioxo 1-imidazolidinyl) 2-(trifluorométhyl) benzonitrile,
- 4-(2,5-dioxo 3-(2-fluoroéthyl) 4-(fluorométhyl) 4-méthyl 1-imidazolidinyl) 2-(trifluorométhyl) benzonitrile,
- 1,5-diméthyl 5-(fluorométhyl) 3-(4-nitro 3-(trifluorométhyl) phényl) 2,4-imidazolidinedione,
- 3-(4-cyano 3-(trifluorométhyl) phényl) 2,4-dioxo 5-(fluorométhyl) 5-méthyl 1-imidazolidinacétonitrile,
- 4-(4,4-bis-(fluorométhyl) 3-méthyl 5-oxo 2-thioxo 1-imidazolidinyl 2-(trifluorométhyl) benzonitrile,
lesdits produits de formule (I) étant sous toutes les formes isomères racémiques, énantiomères et diastéréoisomères possibles, ainsi que les sels d'addition avec les acides minéraux et organiques ou avec les bases minérales et organiques desdits produits de formule (I).

3. Procédé de préparation des produits de formule (I), tels que définis à la revendication 1 ou 2,caractérisé en ce que :
soit l'on fait agir en présence d'une base tertiaire un produit de formule (II) : 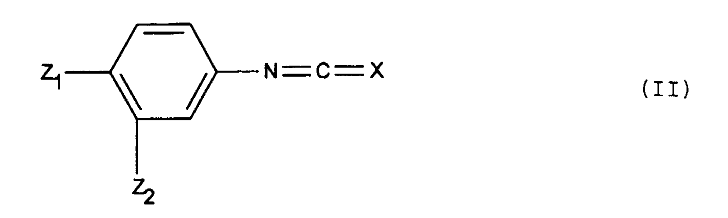 dans laquelle Z₁, Z₂ et X ont la signification indiquée ci-dessus, avec un produit de formule (III) : 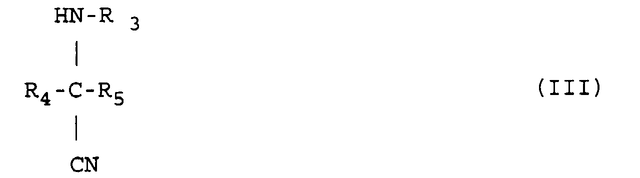 dans laquelle R₃,R₄ et R₅ ont la signification indiquée à la revendication 1 pour obtenir un produit de formule (IV) : 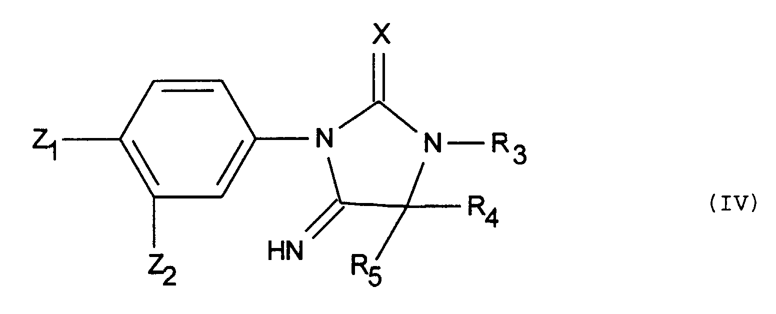 dans laquelle Z₁, Z₂, X, R ₃, R₄ et R₅ ont la signification précédente,
soit l'on fait agir en présence d'une base tertiaire le produit de formule (II), telle que définie ci-dessus, avec un produit de formule (VII) : 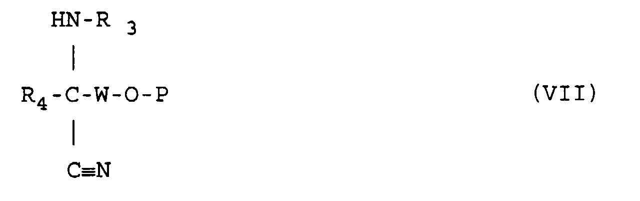 dans laquelle W a la signification indiquée ci-dessus pour R₅ à l'exception du radical alkyle substitué par un radical hydroxyle libres ou substitué par un radical phényle, alkyle renfermant au plus 4 atomes de carbone, ou acyle renfermant au plus 7 atomes de carbone et P représente un groupement protecteur de OH et R ₃ et R₄ ont la signification indiquée ci-dessus, pour obtenir un produit de formule (VIII) : 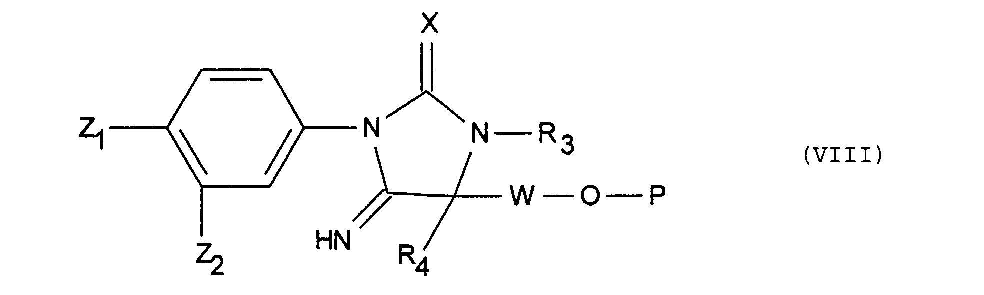 dans laquelle X, Z₁, Z₂, R ₃, R₄, W et P ont la signification indiquée ci-dessus,
produit de formule (VIII) dont si nécessaire et si désiré, l'on peut libérer de OP le radical OH que l'on peut alors si nécessaire et si désiré, estérifier ou transformer en radical halogène,
produits de formules (IV) et (VIII) que, si nécessaire ou si désiré l'on soumet à l'une quelconque ou plusieurs des réactions suivantes, dans un ordre quelconque :
b) réaction d'hydrolyse du groupement >C=NH en fonction carbonyle et le cas échéant transformation du groupement >C=S en groupement >C=O ;
c) réaction de transformation du groupement >C=O en groupement >C=S ;
d) action sur les produits de formule (IV) ou (VIII) dans laquelle R ₃ représente un atome d'hydrogène, et après hydrolyse du groupement >C=NH en fonction carbonyle d'un réactif de formule Hal-R"₃ dans laquelle R"₃ a les valeurs de R ₃ à l'exception de la valeur hydrogène et Hal représente un atome d'halogène pour obtenir des produits de formule (I), tels que définis à la revendication 1 ou 2,
soit l'on fait agir en présence d'une base tertiaire un produit de formule (II) tel que défini ci-dessus, avec un produit de formule (III') : 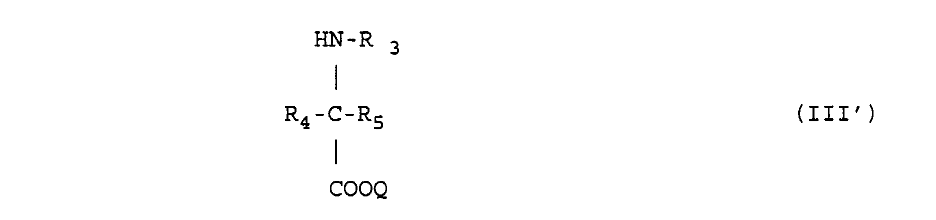 dans laquelle R ₃, R₄ et R₅ ont la signification indiquée ci-dessus et Q représente soit un atome de métal alcalin ou un radical alkyle renfermant de 1 à 6 atomes de carbone, pour obtenir un produit de formule (IVa) : 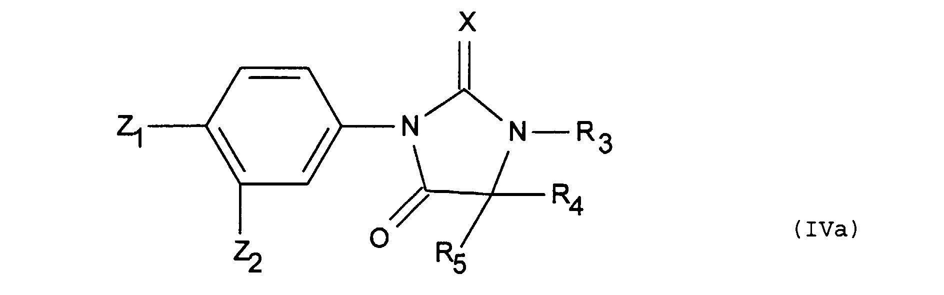 dans laquelle X, Z₁, Z₂, R ₃, R₄ et R₅ ont la signification indiquée ci-dessus, que si désiré l'on soumet à l'une quelconque ou plusieurs des réactions suivantes, dans un ordre quelconque :
b) réaction de transformation du groupement >C=O en groupement >C=S ou le cas échéant du groupement >C=S en groupement >C=O ;
c) action sur les produits de formule (IVa) dans laquelle R ₃ représente un atome d'hydrogène, d'un réactif de formule Hal-R"₃ dans laquelle R"₃ a les valeurs de R ₃ à l'exception de la valeur hydrogène et Hal représente un atome d'halogène pour obtenir des produits de formule (I),tels que définis ci-dessus,
soit l'on fait agir un réactif de formule Hal-R"₃ dans laquelle Hal et R"₃ ont les valeurs indiquées précédemment sur un produit de formule (IV') : 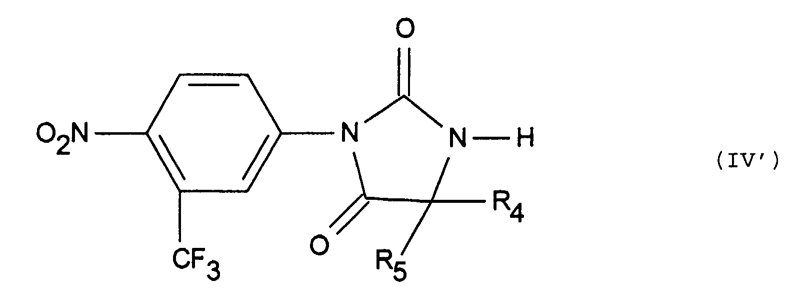 pour obtenir un produit de formule (IV") : 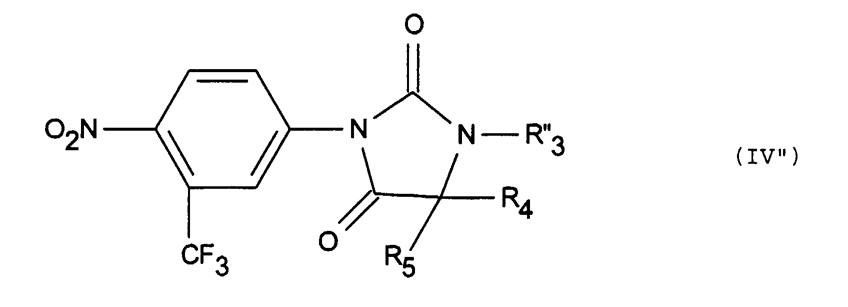 produit de formule (IV") qui représente ou ne représente pas un produit de formule (I) et que, pour obtenir si nécessaire ou si désiré un produit de formule (I), l'on soumet à l'une quelconque ou plusieurs des réactions suivantes dans un ordre quelconque :
b) réaction de transformation du groupement >C=O en groupements >C=S, les dits produits de formule (I) ainsi obtenus étant sous toutes les formes isomères possibles racémiques, énantiomères et diastéréoisomères.

4. A titre de médicaments, les produits de formule (I) tels que définis à la revendication 1 ou 2, ainsi que leurs sels pharmaceutiquement acceptables.

5. Les compositions pharmaceutiques contenant, à titre de principe actif, l'un au moins des médicaments tels que définis à la revendication 4.

## Patentansprüche

1. Produkte der allgemeinen Formel (I): 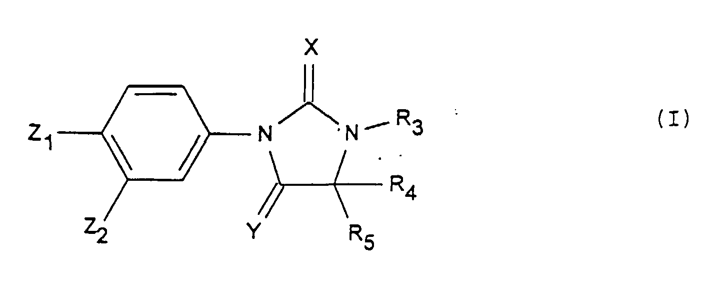 worin
Y ein Sauerstoffatom oder einen Rest NH bedeutet, Z₂ einen Trifluormethylrest bedeutet und Z₁ einen Cyano- oder Nitrorest bedeutet,
X für ein Sauerstoff- oder Schwefelatom steht,
R₃ ein Wasserstoffatom oder einen Alkylrest mit höchstens 4 Kohlenstoffatomen bedeutet, der gegebenenfalls substituiert ist durch einen oder mehrere Reste, ausgewählt unter den Halogenatomen und der Cyanogruppe,
R₄ und R₅ derart sind, daß:
entweder eines Methyl bedeutet und das andere unter den nachstehenden Bedeutungen von R₄ und R₅ ausgewählt ist,
oder R₄ und R₅, identisch oder verschieden, einen linearen oder verzweigten Alkylrest mit höchstens 4 Kohlenstoffatomen, substituiert durch einen Hydroxylrest, gegebenenfalls substituiert durch einen Phenylrest, Alkylrest mit höchstens 4 Kohlenstoffatomen oder Acylrest mit höchstens 7 Kohlenstoffatomen; ein Halogenatom; oder einen Phenylthiorest, gegebenenfalls substituiert durch ein Halogenatom oder einen Hydroxylrest, bedeuten,
mit der Maßgabe, daß bei R₄ und R₅ nicht eines für Methyl und das andere für Hydroxymethyl oder tert.-Butyloxymethyl steht, wenn Z₁ Nitro bedeutet, Z₂ CF₃ bedeutet, R₃ Wasserstoff bedeutet, X Sauerstoff bedeutet und Y Sauerstoff oder NH bedeutet,
wobei die Produkte der Formel (I) in sämtlichen ihrer möglichen isomeren racemischen, enantiomeren und diastereomeren Formen vorliegen können, sowie die Additionssalze mit Mineral- und organischen Säuren oder mit Mineral- und organischen Basen der Produkte der Formel (I).

2. Produkte der Formel (I) gemäß Anspruch 1, mit den folgenden Bezeichnungen:
- 2-(Trifluoromethyl)-4-(4-(hydroxymethyl)-4-methyl-2,5-dioxo-1-imidazolidinyl)-benzonitril,
- 4-(3,4-Dimethyl)-4-(hydroxymethyl)-5-oxo-2-thioxo 1-imidazolidinyl)-2-(trifluoromethyl)-benzonitril,
- 2-(Trifluoromethyl)-4-(4-(hydroxymethyl)-3,4-dimethyl-2,5-dioxo-1-imidazolidinyl)-benzonitril,
- 4-(2,5-Dioxo-3-(2-(fluoroethyl)-4-(hydroxymethyl)-4-methyl-1-imidazolidinyl)-2-trifluoromethyl)-benzonitril,
- 4-(4,4-Bis-(hydroxymethyl)-2,5-dioxo-1-imidazolidinyl)-2-(trifluormethyl)-benzonitril,
- 4-(4-(Fluoromethyl)-3,4-dimethyl)-2,5-dioxo-1-imidazolidinyl-2-(trifluoromethyl)-benzonitril,
- 4-(3,4-Dimethyl)-4-(fluoromethyl)-5-oxo-2-thioxo-1-imidazolidinyl)-2-(trifluoromethyl)-benzonitril,
- 4-(2,5-dioxo-3-(2-fluoroethyl)-4-(fluoromethyl)-4-methyl-1-imidazolidinyl)-2-(trifluoromethyl)-benzonitril,
- 1,5-Dimethyl-5-(fluoromethyl)-3-(4-nitro-3-(trifluoromethyl)-phenyl)-2,4-imidazolidindion,
- 3-(4-Cyano-3-(trifluoromethyl)-phenyl)-2,4-dioxo-5-(fluoromethyl)-5-methyl-1-imidazolidinacetonitril,
- 4-(4,4-Bis-(fluoromethyl)-3-methyl-5-oxo-2-thioxo-1-imidazolidinyl-2-(trifluoromethyl)-benzonitril,
wobei die Produkte der Formel (I) in sämtlichen ihrer möglichen isomeren racemischen, enantiomeren und diastereomeren Formen vorliegen können, sowie die Additionssalze mit Mineral- und organischen Säuren oder mit Mineral- und organischen Basen der Produkte der Formel (I).

3. Verfahren zur Herstellung der Produkte der Formel (I), wie in Anspruch 1 oder 2 definiert, dadurch gekennzeichnet, daß man
entweder in Gegenwart einer tertiären Base ein Produkt der Formel (II): 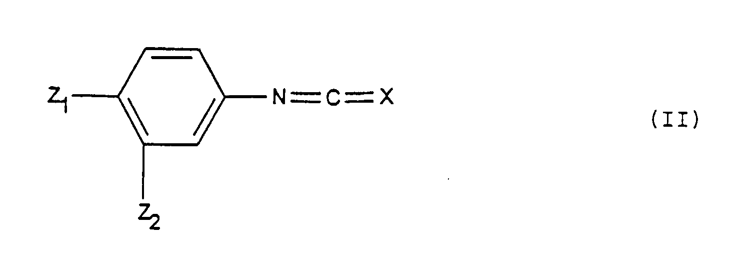 worin Z₁, Z₂ und X die vorstehend angegebene Bedeutung besitzen, mit einem Produkt der Formel (III): 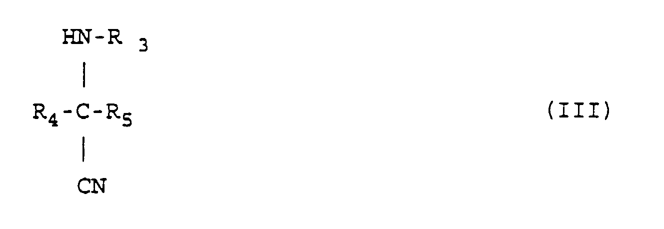 worin R₃, R₄ und R₅ die in Anspruch 1 angegebene Bedeutung besitzen, umsetzt, um zu einem Produkt der Formel (IV): 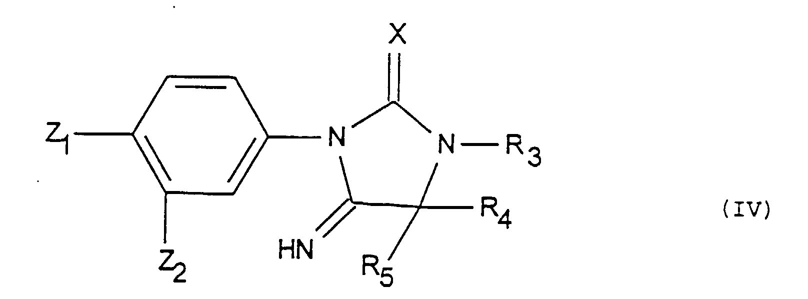 zu gelangen, worin Z₁, Z₂, X, R₃, R₄ und R₅ die vorstehend angegebene Bedeutung besitzen, oder in Gegenwart einer tertiären Base das Produkt der Formel (II), wie vorstehend definiert, mit einem Produkt der Formel (VII): 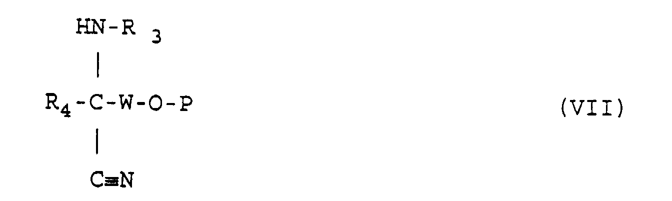 umsetzt, worin W die vorstehend für R₅ angegebene Bedeutung besitzt, mit Ausnahme des Alkylrestes, substituiert durch einen freien Hydroxylrest oder substituiert durch einen Phenylrest, Alkylrest mit höchstens 4 Kohlenstoffatomen oder Acylrest mit höchstens 7 Kohlenstoffatomen, und P eine Schutzgruppe von OH wiedergibt, und R₃ und R₄ die vorstehend angegebene Bedeutung besitzen, um zu einem Produkt der Formel (VIII): 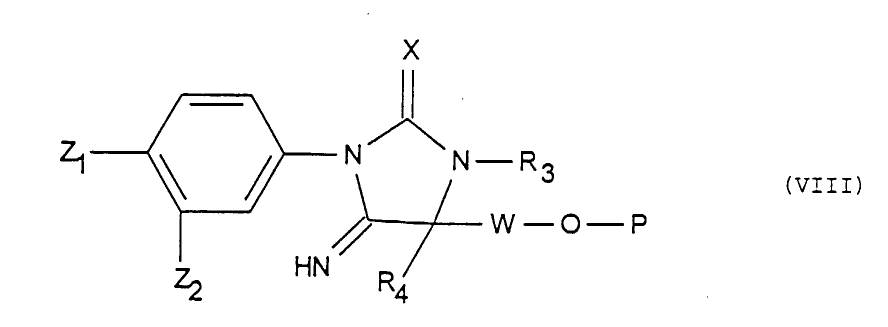 zu gelangen, worin X, Z₁, Z₂, R₃, R₄, W und P die vorstehend angegebene Bedeutung besitzen,
wobei man erforderlichen- und gewünschtenfalls im Produkt der Formel (VIII) aus OP den Rest OH freisetzen kann, welchen man dann erforderlichen- und gewünschtenfalls verestern oder in einen Halogenrest überführen kann,
die Produkte der Formeln (IV) und (VIII) erforderlichen- oder gewünschtenfalls einer oder mehreren der folgenden Reaktionen in beliebiger Reihenfolge unterzieht:
b) Hydrolysereaktion der Gruppe >C=NH in eine Carbonylfunktion und gegenenfalls Umwandlung der Gruppe >C=S in eine Gruppe >C=O;
c) Umwandlungsreaktion der Gruppe >C=O in eine Gruppe >C=S;
d) Umsetzung mit den Produkten der Formel (IV) oder (VIII), worin R₃ ein Wasserstoffatom bedeutet und danach Hydrolyse der Gruppe >C=NH in eine Carbonylfunktion mit einem Reagenz der Formel Hal-R"₃ worin R"₃ die für R₃ angegebenen Bedeutungen, mit Ausnahme der Bedeutung Wasserstoff, besitzt, und Hal ein Halogenatom bedeutet, um zu den Produkten der Formel (I), wie in Anspruch 1 oder 2 definiert, zu gelangen,
oder in Gegenwart einer tertiären Base ein Produkt der Formel (II), wie vorstehend definiert, mit einem Produkt der Formel (III'): 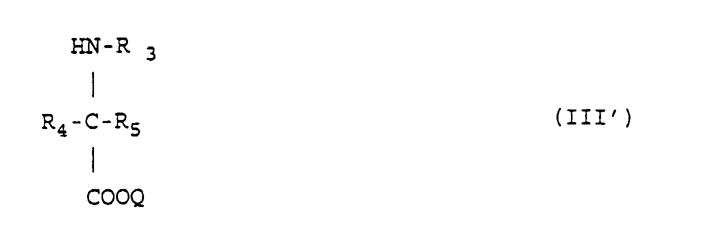 umsetzt, worin R₃, R₄ und R₅ die vorstehend angegebene Bedeutung besitzen und Q entweder ein Alkalimetallatom oder einen Alkylrest mit 1 bis 6 Kohlenstoffatomen bedeutet, um zu einem Produkt der Formel (IVa): 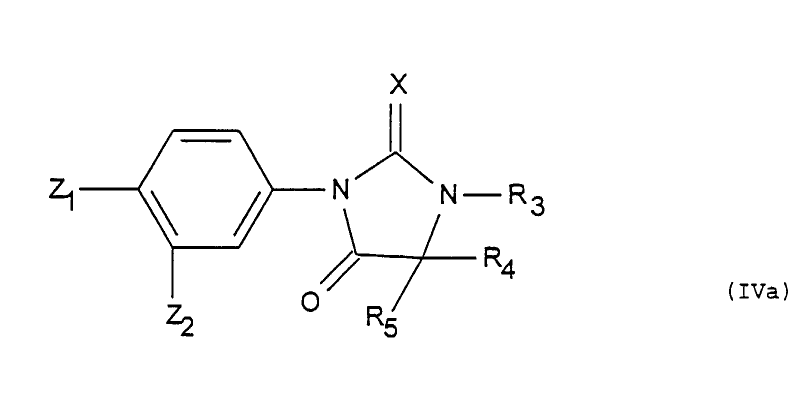 zu gelangen, worin X, Z₁, Z₂, R₃, R₄ und R₅ wie vorstehend definiert sind, welches man gewünschtenfalls einer oder mehreren der folgenden Reaktionen in beliebiger Reihenfolge unterzieht:
b) Umwandlungsreaktion der Gruppe >C=O in die Gruppe >C=S oder gegebenenfalls der Gruppe >C=S in die Gruppe >C=O;
c) Umsetzung der Produkte der Formel (IVa), worin R₃ ein Wasserstoffatom bedeutet, mit einem Reagenz der Formel Hal-R"₃, worin R"₃ die Bedeutung von R₃ mit Ausnahme der Bedeutung Wasserstoff besitzt, und Hal ein Halogenatom wiedergibt, um zu den Produkten der Formel (I), wie vorstehend definiert, zu gelangen,
oder ein Reagenz der Formel Hal-R"₃, worin Hal und R"₃ die vorstehend angegebenen Bedeutungen besitzen, mit einem Produkt der Formel (IV'): 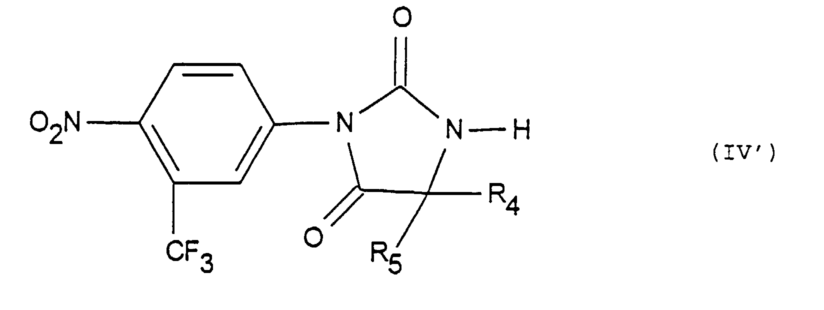 umsetzt, um zu einem Produkt der Formel (IV"): 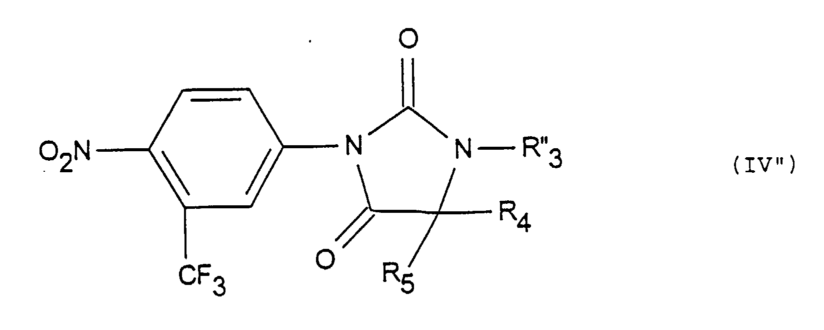 zu gelangen, welches Produkt der Formel (IV") entweder einem Produkt der Formel (I) entspricht oder nicht entspricht, und das man, um erforderlichen- oder gewünschtenfalls ein Produkt der Formel (I) zu erhalten, einer oder mehreren der nachstehenden Reaktionen in beliebiger Reihenfolge unterzieht:
b) Umwandlung der Gruppe >C=O in die Gruppe >C=S, wobei die so erhaltenen Produkte der Formel (I) in sämtlichen ihrer möglichen isomeren racemischen, enantiomeren und diastereomeren Formen vorliegen können.

4. Als Arzneimittel die Produkte der Formel (I) wie in Anspruch 1 oder 2 definiert, sowie deren pharmazeutisch verträgliche Salze.

5. Pharmazeutische Zusammensetzungen, enthaltend als Wirkstoff eines oder mehrere Arzneimittel wie in Anspruch 4 definiert.

## Claims

1. The products of general formula (I): 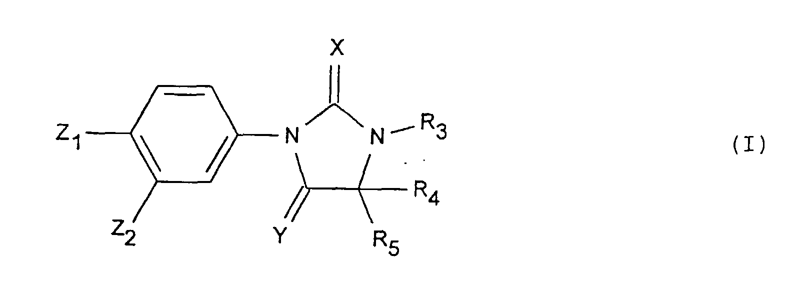 in which:
Y represents an oxygen atom or an NH radical, Z₂ represents a trifluoromethyl radical and Z₁ represents a cyano or nitro radical,
X represents an oxygen or sulphur atom,
R₃ represents a hydrogen atom or an alkyl radical having at most 4 carbon atoms, optionally substituted by one or more radicals chosen from halogen atoms or the cyano radical,
R₄ and R₅ are such that:
either one represents methyl and the other is chosen from the values of R₄ and R₅ hereafter,
or R₄ and R₅, identical or different, represent a linear or branched alkyl radical containing at most 4 carbon atoms substituted by a hydroxyl radical optionally substituted by a phenyl radical, an alkyl radical containing up to 4 carbon atoms or an acyl containing at most 7 carbon atoms; a halogen atom; or a phenylthio radical optionally substituted by a halogen atom or a hydroxyl radical,
it being understood that R4 and R5 do not represent one a methyl and the other a hydroxymethyl or tertiobutyloxymethyl when Z1 represents nitro, Z2 represents CF3, R3 represents hydrogen, X represents oxygen and Y represents oxygen or NH, said products of formula (I) being in all possible racemic, enantiomeric and diastereoisomeric isomer forms, as well as the addition salts with mineral and organic acids or with mineral and organic bases of said products of formula (I).

2. The products of formula (I) as defined in claim 1, the names of which follow:
- 2-(trifluoromethyl) 4-(4-(hydroxymethyl) 4-methyl 2,5-dioxo 1-imidazolidinyl) benzonitrile,
- 4-(3,4-dimethyl) 4-(hydroxymethyl) 5-oxo 2-thioxo 1-imidazolidinyl) 2-(trifluoromethyl) benzonitrile,
- 2-(trifluoromethyl) 4-(4-(hydroxymethyl) 3,4-dimethyl 2,5-dioxo 1-imidazolidinyl) benzonitrile,
- 4-(2,5-dioxo 3-(2-fluoroethyl) 4-(hydroxymethyl) 4-methyl 1-imidazolidinyl) 2-(trifluoromethyl) benzonitrile,
- 4-(4,4-bis(hydroxymethyl)-2,5-dioxo-1-imidazolidinyl)-2-(trifluoromethyl)-benzonitrile,
- 4-(4-(fluoromethyl) 3,4-dimethyl) 2,5-dioxo 1-imidazolidinyl) 2-(trifluoromethyl) benzonitrile,
- 4-(3,4-dimethyl) 4-(fluoromethyl) 5-oxo 2-thioxo 1-imidazolidinyl) 2-(trifluoromethyl) benzonitrile,
- 4-(2,5-dioxo 3-(2-fluoroethyl) 4-(fluoromethyl) 4-methyl 1-imidazolidinyl) 2-(trifluoromethyl) benzonitrile,
- 1,5-dimethyl 5-(fluoromethyl) 3-(4-nitro 3-(trifluoromethyl) phenyl) 2,4-imidazolidinedione,
- 3-(4-cyano 3-(trifluoromethyl) phenyl) 2,4-dioxo 5-(fluoromethyl) 5-methyl 1-imidazolidinacetonitrile,
- 4-(4,4-bis-(fluoromethyl) 3-methyl 5-oxo 2-thioxo 1-imidazolidinyl 2-(trifluoromethyl) benzonitrile,
said products of formula (I) being in all possible racemic, enantiomeric and diastereoisomeric isomer forms, as well as the addition salts with mineral and organic acids or with mineral and organic bases of said products of formula (I).

3. Process for the preparation of the products of formula (I), as defined in claim 1 or 2,characterized in that:
either a product of formula (II): 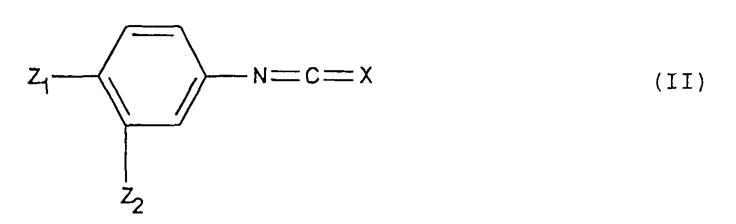 in which Z, Z₂ and X have the meaning indicated above, is reacted in the presence of a tertiary base with a product of formula (III): 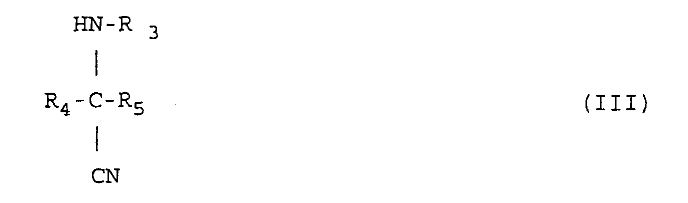 in which R₃, R₄ and R₅ have the meaning indicated in claim 1 in order to obtain a product of formula (IV): 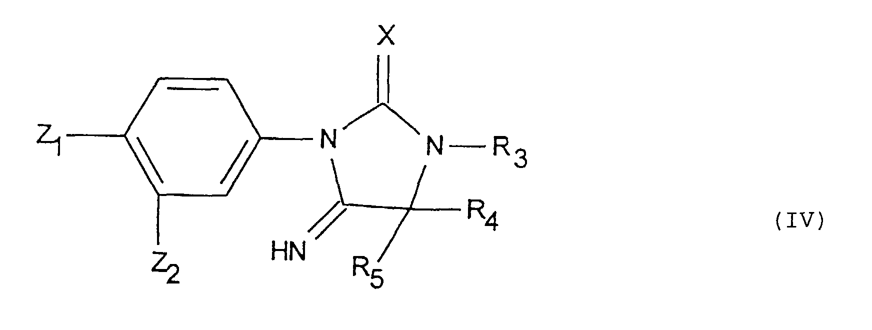 in which Z₁, Z₂, X, R₃, R₄ and R₅ have the previous meaning,
or the product of formula (II), as defined above, is reacted in the presence of a tertiary base with a product of formula (VII): 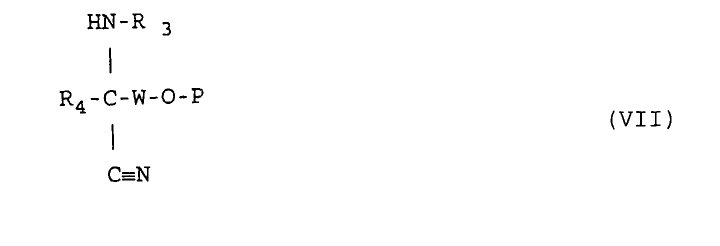 in which W has the meaning indicated above for R₅ with the exception of the alkyl radical substituted by a free hydroxyl radical or substituted by a phenyl, an alkyl radical containing at most 4 carbon atoms or an acyl radical containing at most 7 carbon atoms and P represents a protective group of OH and R₃ and R₄ have the meaning indicated above, in order to obtain a product of formula (VIII): 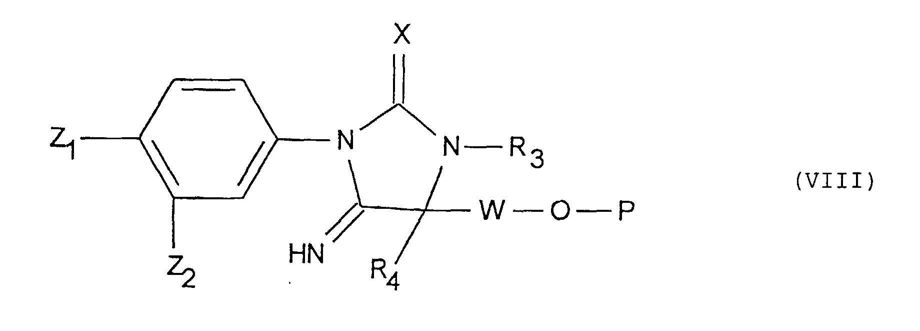 in which X, Z₁, Z₂, R₃, R₄, W and P have the meaning indicated above,
product of formula (VIII) of which if necessary and if desired, the OH radical can be released from OP, which can then if necessary and if desired, be esterified or converted to a halogen radical,
which products of formulae (IV) and (VIII), if necessary or if desired, are subjected to any one or more of the following reactions, in any order:
b) hydrolysis reaction of the >C=NH group to a carbonyl function and if appropriate conversion of the >C=S group to a >C=O group;
c) conversion reaction of the >C=O group to a >C=S group;
d) the action on the products of formula (IV) or (VIII) in which R₃ represents a hydrogen atom, and after hydrolysis of the >C=NH group to a carbonyl function, of a reagent of formula Hal-R"₃ in which R"₃ has the values of R₃ with the exception of the hydrogen value and Hal represents a halogen atom, in order to obtain the products of formula (I), as defined in claim 1 or 2,
or a product of formula (II) as defined above, is reacted in the presence of a tertiary base with a product of formula (III'): 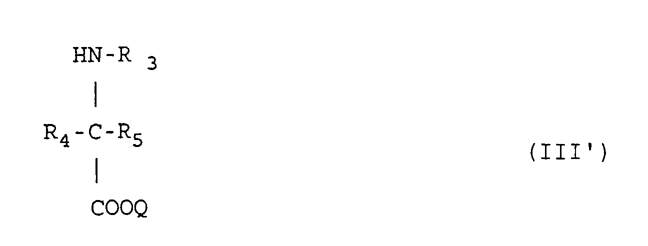 in which R₃, R₄ and R₅ have the meaning indicated above and Q represents either an alkali metal atom or an alkyl radical containing from 1 to 6 carbon atoms, in order to obtain a product of formula (IVa): 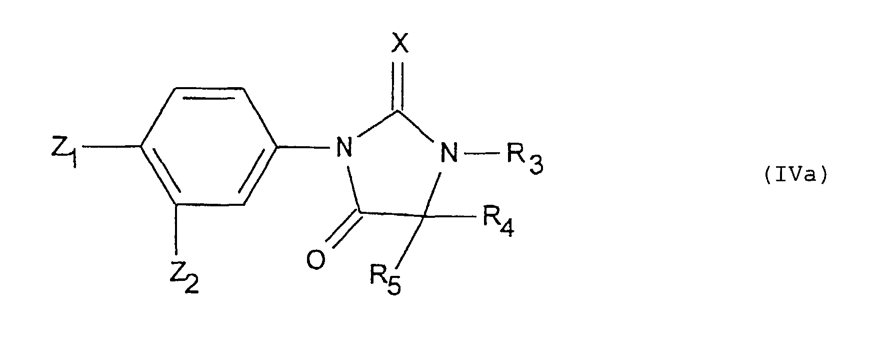 in which X, Z₁, Z₂, R₃, R₄ and R₅ have the meaning indicated above, which if desired is subjected to any one or more of the following reactions, in any order:
b) conversion reaction of the >C=O group to a >C=S group or if appropriate of the >C=S group to a >C=O group;
c) the action on the products of formula (IVa) in which R₃ represents a hydrogen atom, of a reagent of formula Hal-R"₃, in which R"₃ has the values of R₃ with the exception of the hydrogen value and Hal reprocents a halogen atom in order to obtain the products of formula (I), as defined above,
or a reagent of formula Hal-R"₃ in which Hal and R"₃ have the values indicated previously is reacted on a product of formula (IV'): 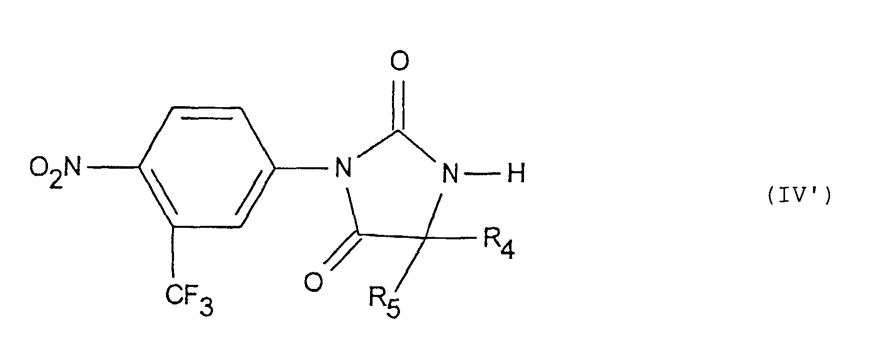 in order to obtain a product of formula (IV"): 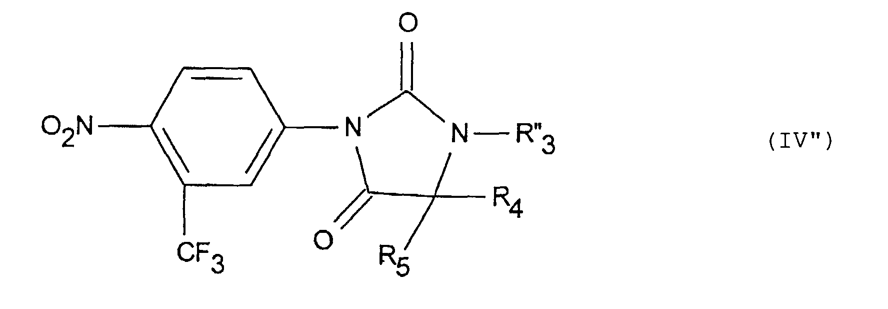 product of formula (IV") which represents or does not represent a product of formula (I) and which, in order to obtain, if necessary or if desired, a product of formula (I), is subjected to any one or more of the following reactions in any order:
b) conversion reaction of the >C=O group to a >C=S group, the said products of formula (I) thus obtained being in all possible racemic, enantiomeric and diastereoisomeric isomer forms.

4. As medicaments the products of formula (I) as defined in claim 1 or 2, as well as their pharmaceutically acceptable salts.

5. The pharmaceutical compositions containing, as active ingredient, at least one of the medicaments as defined in claim 4.
